# EUROPEAN PATENT SPECIFICATION

(11) **EP 0 309 423 B1**
(45) Date of publication and mention of the grant of the patent: **15.06.1994**
(21) Application number: 88830375.7
(22) Date of filing: 19.09.1988
(51) Int. Cl.: C07D 451/12, C07D 453/02, C07D 401/12, C07D 451/14, C07D 403/12, C07D 453/06, C07D 451/04, A61K 31/46, A61K 31/445, A61K 31/435

(54) **New benzimidazoline-2-oxo-1-carboxylic acid derivatives useful as 5-ht receptor antagonists**
Benzimidazolin-2-oxo-1-carboxylsäure-Derivate, verwendbar als Antagonisten von 5-HT-Rezeptoren
Dérivés de l'acide benzimidazoline-2-oxo-1-carboxylique utiles commes antagonistes des 5-HT récepteurs

(30) Priority: 23.09.1987 IT 2199787
(43) Date of publication of application: 29.03.1989
(73) Proprietor: BOEHRINGER INGELHEIM ITALIA S.p.A., 50123 Firenze (IT)
(72) Inventor: Turconi, Marco, Voghera (Pavia) (IT); Donetti, Arturo, Milano (IT); Micheletti, Rosamaria, Milano (IT); Uberti, Annamaria, Milano (IT); Nicola, Massimo, Pavia (IT); Giachetti, Antonio, Milano (IT); Montagna, Ernesto, S.Giuliano Milanese (MI) (IT)
(74) Representative: Aimi, Luciano

(56) References cited:
- EP-A- 0 093 488
- EP-A- 0 247 266
- US-A- 4 250 176

## Description

The present invention relates to novel pharmacologically active benzimidazoline-2-oxo-1-carboxilic acid derivatives, to the process for their preparation and to the pharmaceutical compositions containing them. The new compounds are 5-HT receptor antagonists useful as anti-emetic agents and as gastric prokinetic agents. Serotonin (5-HT) is known to play a major role both in Central Nervous System (CNS) and in Peripheral Nervous System (PNS). Compounds acting as 5-HT receptor antagonists may be effectively used in the prevention or treatment of migraine, cluster headaches and trigeminal neuralgia. They may also be used in the treatment of certain CNS disorders such as anxiety and psychosis. Since 5-HT antagonists may have a beneficial role on gastrointestinal motility a further use of these compounds is in delayed gastric emptying, dyspepsia, flatulence, oesophageal reflux, peptic ulcer, constipation and irritable bowel syndrome. Very recently it has been also discovered that a number of 5-HT antagonists may be particularly useful in the treatment of chemotherapy induced nausea and emesis (J.R. Fozard - Trends in Pharmacological Sciences 8 44, 1987, and references cited therein).

We have now synthetized, and this is the object of the present invention, a novel class of structurally distinct compounds showing specific 5-HT receptor blocking activity which may be useful in the treatment of chemotherapy and radiation induced nausea and emesis and/or of delayed gastric emptying. They may be also of value in the treatment of motion sickness, arrhytmia, migraine, cluster headaches, trigeminal neuralgia, anxiety and psychosis. Moreover they may be used in gastrointestinal motility disorders such as dyspespsia, flatulence, oesophageal reflux, peptic ulcer, constipation, irritable bowel syndrome and hypokinesia.

The compounds, object of the present invention, have the general formula (I) 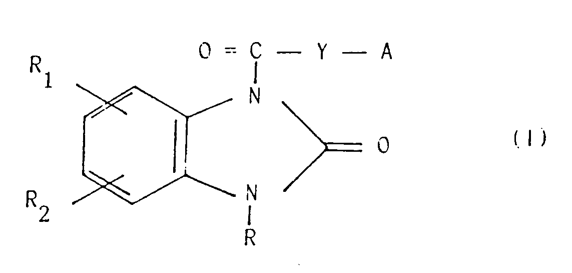
wherein R represents a hydrogen atom, C₁₋₆ alkyl, alkenyl with up to 6 carbon atoms and alkynyl with up to 6 carbon atoms; R₁ and R₂ may be at the same time or not a hydrogen atom, halogen, trifluoromethyl, C₁₋₆ alkyl, C₁₋₆ alkoxy, C₁₋₆ alkythio, C₁₋₆ acyl, carboxyl, C₁₋₆ alkoxycarbonyl, hydroxy, nitro, amino optionally C₁₋₄ alkyl N-mono or di-substituted, C₁₋₆ acylamino, C₁₋₆ alkoxycarbonylamino, carbamoyl optionally C₁₋₄ alkyl N-mono or di-substituted, cyano, C₁₋₆alkylsulphinyl, C₁₋₆ alkylsulphonyl, amino sulphonyl optionally C₁₋₄ alkyl N-mono or di-substituted, C₁₋₄ alkyl N-mono or di-substituted aminosulphonylamino, aminosulphonylamino; Y is oxygen or is N - R₃ in which R₃ is a hydrogen, a C₁₋₆ alkyl or benzyl optionally substituted by one or more C₁₋₆ alkoxy; A is a group selected from: 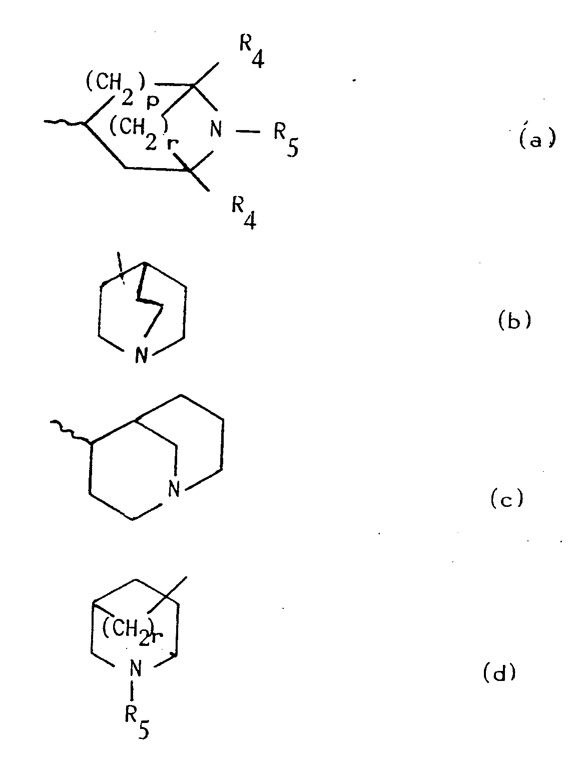
wherein p is 0, 1; r is 0, 1, 2, 3; R₄ is hydrogen atom or a C₁₋₄ alkyl; R₅ is a hydrogen atom, C₁₋₆ alkyl, C₃₋₈ cycloalkyl, C₃₋₈ cycloalkyl C₁₋₄ alkyl, C₁₋₄ alkyl substituted with phenyl or R₅ is a group of formula 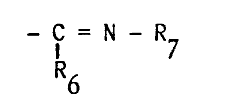
wherein R₆ is hydrogen atom, C₁₋₄ alkyl or an amino group and R₇ is hydrogen atom or C₁₋₆ alkyl.

Document EP-A-0247266 discloses structurally and pharmacologically similar compounds, wherein however no keto function in position 2 and no nitrogen in position 3 of the heterocyclic moiety are present.

For the pharmaceutical use the compounds of general formula (I) are used as such or under the form of tautomers or of physiologically compatible acid addition salts. The term "acid addition salts" includes salts with inorganic or organic acids. Physiologically compatible acids used for the salification include, for example, maleic, citric, tartaric, fumaric, methansulphonic, hydrochloric, hydrobromic, idroiodic, nitric, sulphuric, phosphoric, acetic, benzoic, ascorbic acid.

Physiologically acceptable salts include also quaternary derivatives of compounds of formula (I) obtained by reaction of the above compounds with compounds of formula R₈ - Q wherein R₈ is C₁₋₆ alkyl, phenyl C₁₋₆ alkyl or C₃₋₇ cycloalkyl C₁₋₄ alkyl and Q is a leaving group such as halogen, p-toluensulphonate, mesylate. Preferred R₈ groups are methyl, ethyl, n-propyl, i-propyl, cyclopropylmethyl. Physiologically acceptable salts include also internal salts of compounds of formula (I) such as N-oxides.

The compounds of formula (I) and their physiologically acceptable salts may also exist as physiologically acceptable solvates, such as hydrates, which constitute a further feature of the present invention.

It has to be understood that the carbonyl group in position two of the general formula (I) might exist in its enol form when R is hydrogen and that there are also the tautomers of the amidino derivatives of formula (I) wherein R₅ is a group of formula 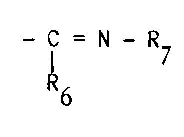
and R₆ and R₇ are as hereinbefore defined. The present invention therefore includes in its scope these tautomeric forms both in term of the compounds and of the manufacturing process.

Some of the compounds of formula (I) according to the present invention contain chiral or prochiral centres and thus may exist in different stereoisomeric forms including enantiomers of (+) and (-) type or mixtures of them. The present invention includes in its scope both the individual isomers and the mixtures thereof.

It has to be understood that, when mixtures of optical isomers are present, they may be separated according to the classic resolution methods based on their different physico-chemical properties, e.g. by fractional crystallization of their acid addition salts with a suitable optically active acid or by the chromatographic separation with a suitable mixture of solvents.

In the present invention the term A of formula (a) means 3-linked 8-azabicyclo[3.2.1]octane or 3-linked 9-azabicyclo[3.3.1]nonane or 2-linked-7-azabicyclo[2.2.1]heptane, 4-linked piperidine, the one of formula (b) means 3 or 4-linked 1-azabicyclo[2.2.2]octane, the one of formula (c) means 4-linked 1-azabicyclo[3.3.1]nonane and the one of formula (d) means 5-linked 2-azabicyclo[2.2.2]octane.

The term halogen means fluorine, chlorine, bromine or iodine. It has also to be understood that in compounds of formula (I) the azabicyclic moieties of group A may be endo or exo substituted.

Compounds of formula (I) containing the pure endo or exo moieties may be prepared starting from the appropriate precursors or by separating mixtures of the endo or exo isomers not stereospecifically synthetized, by conventional methods such as e.g.: chromatography.

The compounds of general formula (I) when R is H may be prepared by reacting a compound of general formula (II) 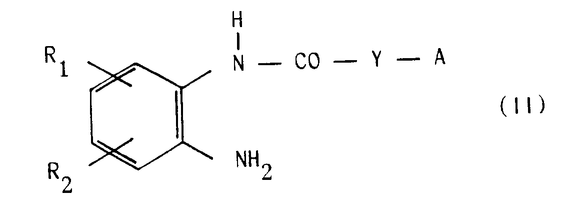
wherein R₁, R₂, Y and A are as hereinbefore defined, with a reactive carbonyl derivative of formula (III) 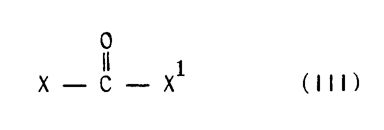
wherein X and X¹ are leaving groups identical or different from each other such as halogen, alogenated alkoxy, alkoxy, heterocycle. Preferred groups are chlorine, trichloromethoxy, methoxy, ethoxy or imidazolyl.

The reaction may be conventionally carried out in aprotic solvents such as benzene, toluene, ethylacetate, acetonitrile, tetrahydrofurane, methylene chloride, chloroform, carbontetrachloride or dimethylformamide at a temperature ranging from 0° to 100°C, preferably at 5°C or at the boiling point of the solvent of choice. The presence of an acid acceptor such as triethylamine may be beneficial in some instances.

Compounds of general formula (II), used as starting materials in the above mentioned process, may be prepared by reducing a compound of formula (IV) 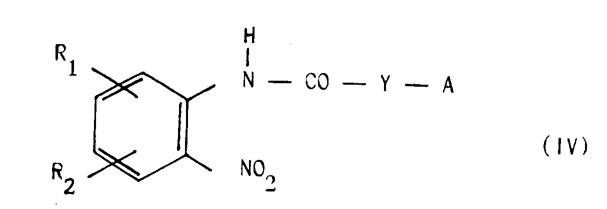
wherein R₁, R₂, Y and A are as hereinbefore defined, with hydrogen or a hydrogen donor such as ammonium formate, cyclohexene, ciclohexadiene, hydrazine. The reduction is preferably carried out with hydrogen in the presence of a suitable catalyst, preferably 5% or 10% Pd/C or Raney nickel in the presence of a suitable solvent such as methanol, ethanol, toluene, water or a mixture of them. The same reduction may be conveniently carried out with iron in acidic medium or in the presence of FeCl₃, with Zn in acetic or hydrochloric acid, with SnCl₂ in hydrochloric acid or with other reducing agents such as titanium trichloride, ferrous sulphate, hydrogen sulphide or its salts, sodium hydrosulphite.

Compounds of general formula (IV) may be prepared by reacting a compound of general formula (V) 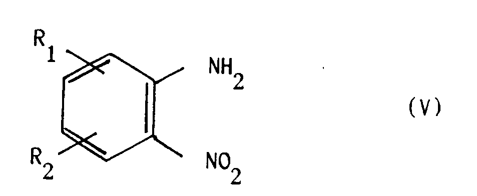
wherein R₁ and R₂ are as hereinbefore defined, with a reactive intermediate of general formula (VI) 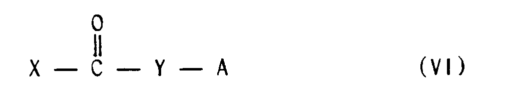
wherein X, Y and A are as hereinbefore defined. The reaction is carried out in an aprotic solvent such as tetrahydrofurane, acetonitrile, chloroform, toluene, chlorobenzene or without solvents, and optionally in the presence of an acid acceptor, preferably in pyridine at a temperature ranging from 20° to 100°C prefetably at 20° or at 80°C.

The compounds of general formula (I), wherein R₁ and R₂ are both hydrogen atoms and R, Y and A are as hereinbefore defined, may conveniently be obtained by reacting a compound of formula (VII) 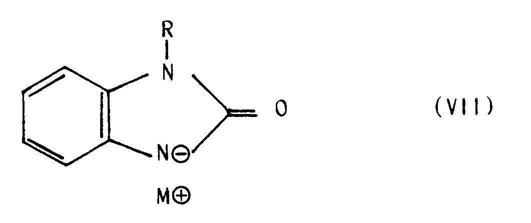
wherein M is a metal atom, such as sodium, potassium or lithium, preferably sodium with a compound of formula (VI). The reaction is preferably carried out in a polar aprotic solvent, such as dimethyl formamide or tetrahydrofurane at a temperature ranging from 0° to 100°C, preferably at room temperature.

Compound (VII) is generated "in situ" from the corresponding hydrogen compounds by means of sodium, potassium, sodium hydride, potassium hydride, potassium tert-butylate, buthyllithium, lithium diisopropylamide, preferably sodium hydride.

The compounds of general formula (I), wherein R₁ and R₂ are both hydrogen atoms R, Y and A are as hereinbefore defined, may be also prepared by reacting a reactive compound of general formula (VIII) 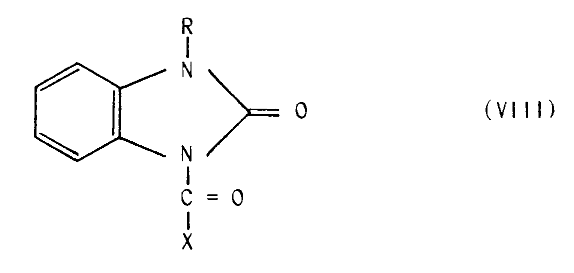
wherein R and X are as hereinbefore defined, with a compound of formula (IX)

Z ― Y ― A (IX)

wherein Z is a hydrogen atom, a metal preferably Li, Na or K, Y and A are as hereinbefore defined.

The reaction is carried out in an aprotic solvent such as tetrahydrofurane, chloroform, acetonitrile, o-dichlorobenzene and optionally in the presence of an acid acceptor such as pyridine or triethylamine, preferably pyridine, at a temperature ranging from 0° to 200°C, preferably at 20° or at 160°C.

It has to be understood that compounds of general formula (I) containing an R, R₁, R₂, R₃ and R₅ group which may give rise to another R, R₁, R₂, R₃ and R₅ group, are useful novel intermediates. Some of these transformations may also occur in the intermediates for compounds of general formula (I).

Some examples of such conversions, which obviously are not exhaustive of all possibilities, are:
1) a nitro group may be transformed into an amino group by reduction
2) an amino group may be transformed into a C₁₋₆ acylamino group by acylation with a suitable carboxylic acid derivative.
3) an amino group may be transformed into a C₁₋₄ alkyl N-mono or di-substituted group by alkylation.
4) an amino group may be transformed into a C₁₋₆ alkoxy carbonyl amino group by reaction with a suitable reactive C₁₋₆ alkyl carbonic acid monoester derivative.
5) a carboxyl group may be transformed into a C₁₋₆ alkoxy carbonyl group, or into a carbamoyl group optionally C₁₋₄ alkyl N-mono or di-substituted by reaction of a suitable reactive carboxylic acid derivative with appropriate alcohols and amines.
6) a carbamoyl group may be transformed into a cyano group by dehydration
7) a C₁₋₆ alkyl thio or a C₁₋₆ alkyl sulphinyl group may be transformed into a C₁₋₆ alkyl sulphinyl or a C₁₋₆ alkylsulphonyl group by oxidation
8) an aromatic hydrogen group may be transformed into a nitro group by nitration
9) a hydrogen group may be transformed into a halogen group by halogenation
10) a secondary amide group, optionally conjugated with other carboxamidic moieties, may be transformed into a C₁₋₆ N-alkyl tertiary amide group by alkylation
11) a secondary amino group may be transformed into an amidino derivative by reaction with appropriate reactive compounds such as e.g. ethyl formimidate, ethyl acetimidate or cyanamide
12) a tertiary amino group may be transformed into a quaternary ammonium derivative by reaction with a suitable alkilating agent such as methyl bromide or methyl iodide.
13) A tertiary amidic group optionally conjugated with other carboxamidic moieties may be transformed into a secondary amidic group by removing an optionally C₁₋₆ alkoxy substituted benzyl.

These transformations are well known to any expert of the branch.

The compounds of general formula (I) prepared according to the process as above described may optionally be converted with inorganic or organic acids into the corresponding physiologically compatible acid addition salts, for example, by conventional methods such as by reacting the compound as bases with a solution of the corresponding acid in a suitable solvent. Particularly preferred acids include for example hydrochloric, hydrobromic, sulphuric, acetic, citric, tartaric acids.

Preferred groups of the compounds, according to the present invention, for their better activity as 5-HT receptor blocking agents, are the ones formed by the compounds of general formula (I) wherein:
- A is endo-8-methyl-8-azabicyclo[3.2.1]oct-3-yl, R₁ and R₂ are H, R is H or CH₃ and Y is oxygen or a NH group.
- A is 1-azabicyclo[2.2.2]oct-3-yl, R₁ and R₂ are H, R is H or CH₃ and Y is oxygen or an NH group.
- A is endo-9-methyl-9-azabicyclo[3.3.1]non-3-yl, R₁ and R₂ are H, R is H or CH₃ and Y is oxygen or an NH group.
- A is endo-1-azabicyclo[3.3.1]non-4-yl, R₁ and R₂ are H, R is H or CH₃ and Y is oxygen or a NH group.

As already mentioned hereinbefore the new compounds of formula (I), according to the present invention, have interesting pharmacological properties owing to their ability to antagonize the physiological 5-HT effects in warm-blooded animals. Therefore the new compounds are commercially viable in the prevention and in the treatment of disorders wherein 5-HT receptors are involved such as chemotherapy or radiation induced nausea and emesis, and delayed gastric empting.

The following tests show that the compounds according to the present invention have favourable characteristics in this respect.

### PHARMACOLOGY

### Bezold-Jarisch reflex in anaesthetized rats

Rats (250-275 g) were anaesthetized with urethane (1.25 g/kg ip.).

Blood pressure was recorded from the left femoral artery by means of a pressure transducer (Statham) and heart rate was recorded by feeding a cardiotachometer with the blood pressure signal.

The Bezold-Jarisch effect was elicited by rapid bolus intravenous injection of 5-HT (20 »g/kg).

Increasing doses of antagonists were injected 5 min before 5-HT to evaluate their effect on the initial abrupt cardiac slowing and associated fall in blood pressure resulting from the reflex vagal stimulation. In other experiments, the right vagus nerve was stimulated with platinum electrodes at 10 V, 10 Hz, 2 msec, to evoke bradycardia (Grass 248 stimulator). ED₅₀ values were calculated by linear regression analysis of the data expressed as percentage inhibition.

The obtained potency of two compounds object of the present invention is shown below:

| | Bradycardia ED₅₀(»g/kg⁻¹, i.v.) | Hypotension ED₅₀(»g/kg⁻¹, i.v.) |
|---|---|---|
| Compound 26 | 0.3 | 0.4 |
| Compound 27 | 0.35 | 0.51 |
| Compound 28 | 1.0 | 1.5 |
| Compound 31 | 0.49 | 1.97 |

### Guinea pig ileum longitudinal muscle-myenteric plexus

Male guinea pigs (Dunkin Hartley, 450-550 g) were killed by cervical dislocation. A 2 cm segment of distal ileum, removed about 10 cm proximal to the caecum was suspended under 0.5 g tension in a 10 ml organ bath containing Tyrode solution (mM: NaCl 137; KCl 2.68; CaCl₂ 1.82; NaHCO₃ 5.9; MgCl₂ 1; NaH₂PO₄ 0.42; glucose 5.6) oxygenated with 95% O₂ 5% CO₂, at 37°C. Responses were registered with an isotonic transducer on a polygraph (Basile).

Electrical field stimulation (EFS) was performed with bipolar platinum electrodes, with 0.5 msec pulses at 0.1 Hz frequency, supramaximal voltage. When contractions had stabilized, cumulative concentration-response curves were constructed for the compounds under investigation, by adding increasing concentrations at 5 min intervals.

The effect of compounds on EFS evoked contractions was evaluated as percentage of the contraction height measured before addition of compounds.

The compounds, object of the present invention, strengthened the contractions induced by electrical stimulation in the guinea pig ileum in the concentration range 10⁻¹⁰ - 10⁻⁸ M, while having no effect on muscle tone.

According to a further feature of the present invention there are provided pharmaceutical compositions comprising as active ingredient at least one compound of formula (I), as hereinbefore defined, or a physiologically compatible acid addition salt thereof in association with pharmaceutical carriers or excipients..

For pharmaceutical administration the compounds of general formula (I) and their physiologically compatible acid addition salts may be incorporated into the conventional pharmaceutical preparations in either solid or liquid form. The compositions may, for example, be presented in a form suitable for oral, rectal or parenteral administration. Preferred forms include, for example, capsules, tablets, coated tablets, ampoules, suppositories and oral drops.

The active ingredient may be incorporated in excipients or carrier conventionally used in pharmaceutical compositions such as, for example, talc, gum arabic, lactose, gelatine, magnesium stearate, corn starch, aqueous or non-aqueous vehicles, polyvinylpirrolidone, mannitol, semisynthetic glicerides of fatty acids, sorbitol, propylene glycol, citric acid, sodium citrate.

The compositions are advantageously formulated at dosage units, each dosage unit being adapted to supply a single dose of the active ingredient. Each dosage unit may conveniently contain from 50 mg to 1000 mg and preferably from 100 mg to 500 mg of the above ingredient.

The following examples illustrate some of the new compounds according to the present invention.

### Example 1

### Endo-8-methyl-8-azabicyclo[3.2.1]oct-3-yl chloroformate hydrochloride

43 g of endo-8-methyl-8-azabicyclo[3.2.1]octan-3-ol hydrochloride were suspended in 400 ml of acetonitrile and 62.2 g of trichloromethyl chloroformate dissolved in 40 ml of acetonitrile were added at 0°C. The reaction mixture was stirred at room temperature for 24 hrs obtaining a clear solution, which was concentrated to dryness and the residue was triturated with diethyl ether. 56.8 g of a white solid were obtained.
M.p. 134-136°C (dec.).

Similarly were obtained:
1-azabicyclo[2.2.2]oct-3-yl chloroformate hydrochloride. M.p. 130-132°C.
Endo-8-ethyl-8-azabicyclo[3.2.1]oct-3-yl chloroformate hydrochloride.
M.p. 145-147°C.
1,2,3-trimethyl-piperidin-4-yl chloroformate hydrochloride.
M.p. 141-143°C.
Endo-8-phenylmethyl-8-azabicyclo[3.2.1]oct-3-yl chloroformate hydrochloride. M.p. 152-153°C.
Exo-8-methyl-8-azabicyclo[3.2.1]oct-3-yl chloroformate hydrochloride.
M.p. 137-140°C.
Endo-9-methyl-9-azabicyclo[3.3.1]non-3-yl chloroformate hydrochloride.
M.p. 117-120°C.
Exo-9-methyl-9-azabicyclo[3.3.1]non-3-yl chloroformate hydrochloride.
M.p. 152-153°C.

### Example 2

### 2,3-Dihydro-2-oxo-1H-benzimidazole-1-carbonyl chloride

It was prepared by suspending 5 g of 2,3-dihydro-1H-benzimidazole-2-one in 200 ml of distilled tetrahydrofuran and by adding 13.5 ml of trichloromethylchloroformate. The reaction mixture was refluxed for 3 hrs until a clear solution was obtained. After cooling the separated solid was removed by filtration and after concentration to dryness of the mother liquors 6.5 g of the title compound were obtained.
M.p. 188-190°C (dec.).

### Example 3

### Endo-3-[(2,4-dimethoxyphenyl) methyl] amino-9-methyl-9-azabicyclo[3.3.1] nonane

a) 2g of exo-9-methyl-9-azabicyclo[3.3.1]nonan-3-ol were dissolved in methylene chloride (40 ml) and to the solution cooled at 0°C 3.8 ml of thionyl chloride were added under stirring. After refluxing for 4 hours the reaction mixture was evaporated to dryness. The residue was taken up in water, made basic and extracted with methylene chloride. After evaporation of the solvent 2 g of a crude oil were obtained and after purification by flash-chromatography on silica-gel (eluent methylene chloride-methanol-32% ammonium hydroxyde 97:3:0.3) 0.8 g of endo-3-chloro-9-methyl-9-azabicyclo-[3.3.1]nonane were obtained.
   M.p. 177-178°C.
b) A solution of endo-3-chloro-9-methyl-9-azabicyclo[3.3.1]nonane (0.5 g) and 2,4-dimethoxybenzylamine (0.62 g) in absolute ethanol (50 ml) was refluxed for 4 hrs. After cooling the crude product, obtained by evaporation of the solvent, was purified by flash-chromatography on silicagel (eluent methylene chloride-methanol-32% ammonium hydroxide 90:10:1) 0.35 g of oil were obtained. By using known methods 0.24 g of the title compound as dihydrochloride were obtained. M.p. 173-175°C.

### Example 4

### Endo-3-methylamino-9-methyl-9-azabicyclo[3.3.1]nonane

10 ml of a solution of 33 % ethanolic methylamine were added to a solution of 9-methyl-9-azabicyclo[3.3.1]nonan-3-one (1.7 g) in ethanol (30 ml).

The reaction mixture was left in a closed vessel for 4 days, then it was hydrogenated at room temperature and at atmosphere pressure in the presence of previously reduced PtO₂ (0.2 g) and ammonium acetate (1.0 g).

When the absorption of hydrogen was finished, the catalyst was removed by filtration. The reaction mixture was concentrated to dryness, taken up in water, made basic with sodium hydroxide and extracted with ethyl acetate. After drying over MgSO₄ the organic phase gave, as a residue, 1.2 g of the title compound as a yellow oil which was used as such for the next reaction.

### Example 5

### N-(2-amino-5-nitrophenyl) (endo-8-methyl-8-azabicyclo[3.2.1]otc-3-yl-carbamate

Endo-8-methyl-8-azabicyclo-[3.2.1]oct-3-yl chloroformate hydrochloride (7.85 g) was added portionwise, under stirring at room temperature, to a solution of 5 g of 4-nitro-1,2-phenylendiamine in pyridine (70 ml).

The reaction mixture was stirred at the same temperature for 1 hr, evaporated to dryness, taken up in water and made acidic with HCl. The aqueous phase was washed with ethyl acetate and made basic with sodium hydroxide. A solid separated which was removed by filtration. 4.8 g of the title compound were obtained. M.p. 75-77°C.

### Example 6

### Exo-2-methyl-2-azabicyclo[2.2.2]octan-5-ol

The product was obtained according to R.F. Borne - J. Med. Chem. 16, 853-856 (1973). In that paper the compound was identified as "cis".

### Example 7

### Endo-2-methyl-2-azabicyclo[2.2.2]octan-5-ol

The product was obtained according to R.F. Borne - J. Med. Chem. 16, 853-856 (1973). In that paper the compound was identified as "trans".

### Example 8

### Endo-7-methyl-7-azabicyclo[2.2.1]eptan-2-ol

The product was obtained according to J.R. Phister - J. Pharm. Sci. 74, 208 (1985).

### Example 9

### N-(2-aminophenyl) (endo-8-ethyl-8-azabicyclo[3.2.1]oct-3-yl)carbamic acid ester

a) To a solution of 2-nitroaniline (5.0 g) in dry pyridine (75 ml) endo-8-methyl-8-azabicyclo[3.2.1]oct-3-yl chloroformate hydrochloride (8.7 g) was added portionwise under stirring at room temperature. Once the initial exothermic reaction had subsided the reaction mixture was heated to 80°C and stirred for 4 hrs. After cooling the pure N-(2-nitrophenyl)(endo-8-methyl-8-azabicyclo[3.2.1]oct-3-yl)carbamic acid ester, hydrochloride was recovered by filtration. 6.5 g. M.p. > 250°C
   IR (cm⁻¹) nujol : 1720, 1605, 1590, 1520
   Starting from the proper chloroformate hydrochlorides and the proper 2-nitroaniline derivatives the following compounds were also obtained:
   N-(4-methoxy-2-nitrophenyl)(endo-8-methyl-8-azabicyclo[3.2.1]oct-3-yl)carbamic acid ester, hydrochloride. M.p. 248°-250°C
   N-(4-methyl-2-nitrophenyl)(endo-8-methyl-8-azabicyclo[3.2.1]oct-3-yl)carbamic acid ester, hydrochloride. M.p. > 250°C.
   N-(2-nitrophenyl)(1-azabicyclo[2.2.2]oct-3-yl)carbamic acid ester, hydrochloride. M.p. > 250°C.
   N-(2-nitrophenyl)(exo-8-methyl-8-azabicyclo[3.2.1]oct-3-yl)carbamic acid ester, hydrochloride. M.p. 68-69°C.
   N-(2-nitrophenyl)(1-methylpiperidin-4-yl)carbamic acid ester. M.p. 87-89°C.
   N-(2-nitrophenyl)(1,2,6-trimethylpiperidin-4-yl)carbamic acid ester.
   M.p. 130-132°C.
   N-(2-nitrophenyl)(endo-8-ethyl-8-azabicyclo[3.2.1]oct-3-yl)carbamic acid ester, hydrochloride. M.p.>260°C.
   N-(4-chloro-2-nitrophenyl)(endo-8-methyl-8-azabicyclo[3.2.1]oct-3-yl)carbamic acid ester. M.p. 120-122°C.
   N-(5-fluoro-2-nitrophenyl)(endo-8-methyl-8-azabicyclo[3.2.1]oct-3-yl) carbamic acid ester, hydrochloride. M.p. 257-258°C.
   N-(4,5-dimethyl-2-nitrophenyl)(endo-8-methyl-8-azabicyclo[3.2.1]oct-3-yl) carbamic acid ester. M.p. 138-139°C.
   N-(4-fluor-2-nitrophenyl)(endo-8-methyl-8-azabicyclo[3.2.1]oct-3-yl) carbamic acid ester, hydrochloride. M.p. 255-256°C.
   N-(6-methyl-2-nitrophenyl)(endo-8-methyl-8-azabicyclo[3.2.1]oct-3-yl) carbamic acid ester, hydrochloride. M. p. >260°C.
   N- (4-trifluoromethyl-2-nitrophenyl)(endo-8-methyl-8-azabicyclo[3.2.1] oct-3-yl)carbamic acid ester. M.p. 114-115°C.
   N-(5-methoxy-2-nitrophenyl)(endo-8-methyl-8-azabicyclo[3.2.1]oct-3-yl) carbamic acid ester. M.p. 123-124°C.
   N-(5-acetyl-2-nitrophenyl)(endo-8-methyl-8-azabicyclo[3.2.1]oct-3-yl) carbamic acid ester. M.p. 106-108°C.
   N-(2-nitrophenyl)(endo-8-phenylmethyl-8-azabicyclo[3.2.1]oct-3-yl) carbamic acid ester, hydrochloride. M.p. 205-207°C.
b) A solution of N-(2-nitrophenyl)(endo-8-methyl-8-azabicyclo[3.2.1]oct-3-yl)carbamic acid ester, hydrochloride (6.5 g) in 70% aqueous ethanol (200 ml) was hydrogenated at room temperature and atmospheric pressure in the presence of 10% Pd/C (0.3 g). After the teoretical absorption the reaction mixture was filtered and concentrated to dryness. The residue was taken up in acidic water and the aqueous phase was washed with diethyl ether. The aqueous phase was then made basic and extracted with ethyl acetate; the organic extracts were dried over MgSO₄ and concentrated to dryness. 4.4 g of pure title compound were obtained from diisopropyl ether. M.p. 155°-157°C.
   IR (cm⁻¹) nujol: 3420, 3260, 1680, 1605, 1590, 1540.
   Similarly and using, according to the cases, an appropriate catalyst or general methods of chemical reduction, the following compounds were obtained:
   N-(4-methoxy-2-aminophenyl)(endo-8-methyl-8-azabicyclo[3.2.1]oct-3-yl)carbamic acid ester. M.p. 118°-120°C.
   N-(4-methyl-2-aminophenyl)(endo-8-methyl-8-azabicyclo[3.2.1]-oct-3-yl)carbamic acid ester. M.p. 147°-149°C.
   N-(2-aminophenyl)(1-azabicyclo[2.2.2]oct-3-yl)carbamic acid ester. M.p. 165°-167°C.
   N-(2-aminophenyl)(exo-8-methyl-8-azabicyclo[3.2.1]oct-3-yl)carbamic acid ester. M.p. 156-158°C.
   N-(2-aminophenyl)(1-methylpiperidin-4-yl)carbamic acid ester.
   M.p. 153-155°C.
   N-(2-aminophenyl)(1,2,6-trimethylpiperidin-4-yl)carbamic acid ester.
   M.p. 190-192°C.
   N-(2-aminophenyl)(endo-8-methyl-8-azabicyclo[3.2.1]oct-3-yl) carbamic acid ester. M.p. 136-138°C.
   N-(2-amino-4-chlorophenyl)(endo-8-methyl-8-azabicyclo[3.2.1]oct-3-yl) carbamic acid ester 130-133°C.
   N-(2-amino-5-fluorophenyl)(endo-8-methyl-8-azabicyclo[3.2.1]oct-3-yl) carbamic acid ester. M.p. 180-181°C.
   N-(2-amino-4,5-dimethylphenyl)(endo-8-methyl-8-azabicyclo[3.2.1]oct-3-yl) carbamic acid ester. M.p. 142-143°C.
   N-(2-amino-4-fluorophenyl)(endo-8-methyl-8-azabicyclo[3.2.1]oct-3-yl) carbamic acid ester. M.p. 171-172°C.
   N-(2-amino-6-methylphenyl)(endo-8-methyl-8-azabicyclo[3.2.1]oct-3-yl) carbamic acid ester. M.p. 118-120°C.
   N-(2-amino-4-trifluoromethylphenyl)(endo-8-methyl-8-azabicyclo[3.2.1] oct-3-yl) carbamic acid ester. M.p. 141-142°C.
   N-(2-amino-5-methoxyphenyl)(endo-8-methyl-8-azabicyclo[3.2.1]oct-3-yl) carbamic acid ester. M.p. 144-145°C.
   N-(5-acetyl-2-aminophenyl)(endo-8-methyl-8-azabicyclo[3.2.1]oct-3-yl) carbamic acid ester. M.p. 118-121°C.
   N-(2-aminophenyl)(endo-8-methyl-8-azabicyclo[3.2.1]oct-3-yl)carbamic acid ester. M.p. 153-155°C.

### Example 10

### 2,3-Dihydro-2-oxo-1H-benzimidazole-1-carboxylic acid (endo-8-methyl-8-azabicyclo[3.2.1]oct-3-yl)ester

### (Compound 1)

A solution of N-(2-aminophenyl)(endo-8-methyl-8-azabicyclo[3.2.1] oct-3-yl)carbamic acid ester (4.14 g) and triethylamine (2.5 ml) in dry methylene chloride (65 ml) was slowly added dropwise into a solution of trichloromethyl chloroformate (1.99 ml) in the same solvent (20 ml) at 5°C under stirring. When the addition was over (60 min) the temperature was allowed to reach 25°C while stirring was continued for another 60 min. Acidic water was then added and the organic phase was discarded; the aqueous phase was made basic and extracted with methylene chloride.

After evaporation of the solvent the crude product was obtained and cristallized from acetonitrile. 2.2 g.
M.p. 191°-192°C.
MS (C.l.): 302 m/e [M + H]⁺
IR (cm⁻¹) nujol: 1760, 1720

| Analysis | | | | |
|---|---|---|---|---|
| C₁₆H₁₉N₃O₃ | Found % | C 63.40 | H 6.42 | N 13.76 |
| | Calc. % | C 63.77 | H 6.36 | N 13.95 |

The hydrochloride salt was also prepared. M.p. 260°-261°C (CH₃CN)
Similarly were prepared:

### 5-methoxy-2,3-dihydro-2-oxo-1H-benzimidazole-1-carboxylic acid (endo-8-methyl-8-azabicyclo[3.2.1]oct-3-yl)ester

### (Compound 2)

M.p. 177°-178°C
MS (C.l.) 332 m/e [M + H]

| Analysis | | | | |
|---|---|---|---|---|
| C₁₇H₂₁N₃O₄ | Found % | C 61.45 | H 6.35 | N 12.72 |
| | Calc. % | C 61.62 | H 6.39 | N 12.68 |

### 5-methyl-2,3-dihydro-2-oxo-1H-benzimidazole-1-carboxylic acid (endo-8-methyl-8-azabicyclo[3.2.1]oct-3-yl)ester

### (Compound 3)

M.p. 187°-189°C
MS (C.l.): 316 m/e [M + H]⁺

| Analysis | | | | |
|---|---|---|---|---|
| C₁₇H₂₁N₃O₃ | Found % | C 64.23 | H 6.73 | N 13.39 |
| | Calc. % | C 64.74 | H 6.71 | N 13.33 |

### 2,3-dihydro-2-oxo-1H-benzimidazole-1-carboxylic acid (1-azabicyclo[2.2.2] oct-3-yl) ester, hydrochloride

### (Compound 4)

M.p. 260°C.
MS (C.l.): 288 m/e [M + H]⁺
IR (cm⁻¹): 1760, 1720 broad

| Analysis | | | | |
|---|---|---|---|---|
| C₁₅H₁₇N₃O₃·HCl | Found % | C 55.17 | H 5.62 | N 12.75 |
| | Calc. % | C 55.64 | H 5.60 | N 12.98 |

### 2,3-dihydro-2-oxo-1H-benzimidazole-1-carboxylic acid (exo-8-methyl-8-azabicyclo[3.2.1]oct-3-yl) ester

### (Compound 5)

Hydrochloride. M.p. 255-256°C

| Analysis | | | | |
|---|---|---|---|---|
| C₁₆H₁₉N₃O₃·HCl | Found % | C 56.53 | H 6.01 | N 12.21 |
| | Calc. % | C 56.83 | H 5.97 | N 12.44 |

### 2,3-dihydro-2-oxo-1H-benzimidazole-1-carboxylic acid (1-methylpiperidin-4-yl) ester.

### (Compound 6)

Hydrochloride. M.p. 219-220°C.

| Analysis | | | | |
|---|---|---|---|---|
| C₁₄H₁₇N₃O₃ ·HCl | Found % | C 56.55 | H 5.73 | N 12.15 |
| | Calc. % | C 53.93 | H 5.82 | N 13.47 |

### 2,3-dihydro-2-oxo-1H-benzimidazole-1-carboxylic acid (1,2,6-trimethylpiperidin-4-yl) ester

### (Compound 7)

Hydrochloride. M.p. 250-252°C

| Analysis | | | | |
|---|---|---|---|---|
| C₁₆H₂₁N₃O₃·HCl | Found % | C 56.45 | H 6.61 | N 12.05 |
| | Calc. % | C 56.55 | H 6.53 | N 12.37 |

### 2,3-dihydro-2-oxo-1H-benzimidazole-1-carboxylic acid (endo-8-ethyl-8-azabicyclo[3.2.1]oct-3-yl) ester

### (Compound 8)

Hydrochloride. M.p. 268-270°C.

| Analysis | | | | |
|---|---|---|---|---|
| C₁₇H₂₁N₃O₃·HCl | Found % | C 57.65 | H 6.37 | N 11.72 |
| | Calc. % | C 58.03 | H 6.30 | N 11.94 |

### 5-chloro-2,3-dihydro-2-oxo-1H-benzimidazole-1-carboxylic acid (endo-8-methyl-8-azabicyclo[3.2.1]oct-3yl) ester

### (Compound 9)

Hydrochloride. M.p. 236-238°C.

| Analysis | | | | |
|---|---|---|---|---|
| C₁₆H₁₈ClN₃O₃·HCl | Found % | C 50.93 | H 5.21 | N 11.03 |
| | Calc. % | C 51.62 | H 5.14 | N 11.29 |

### 6-fluoro-2,3-dihydro-2-oxo-1H-benzimidazole-1-carboxylic acid (endo-8-methyl-8-azabicyclo[3.2.1]oct-3-yl) ester

### (Compound 10)

Hydrochloride. M.p. 261-263°C.

| Analysis | | | | |
|---|---|---|---|---|
| C₁₆H₁₈FN₃O₃·HCl | Found % | C 53.65 | H 5.45 | N 11.69 |
| | Calc. % | C 54.00 | H 5.38 | N 11.81 |

### 5,6-dimethyl-2,3-dihydro-2-oxo-1H-benzimidazole-1-carboxylic acid (endo-8-methyl-8-azabicyclo[3.2.1]oct-3-yl) ester

### (Compound 11)

Hydrochloride. M.p. > 260°C.

| Analysis | | | | |
|---|---|---|---|---|
| C₁₈H₂₃N₃O₃·HCl | Found % | C 58.51 | H 6.65 | N 11.27 |
| | Calc. % | C 59.09 | H 6.61 | N 11.49 |

### 5-fluoro-2,3-dihydo-2-oxo-1H-benzimidazole-1-carboxylic acid (endo-8-methyl-8-azabicyclo[3.2.1]oct-3-yl) ester

### (Compound 12)

Hydrochloride. M.p. 257-258°C.

| Analysis | | | | |
|---|---|---|---|---|
| C₁₆H₁₈FN₃O₃·HCl | Found % | C 53.89 | H 5.41 | N 11.71 |
| | Calc. % | C 54.00 | H 5.38 | N 11.81 |

### 6-acetyl-2,3-dihydro-2-oxo-1H-benzimidazole-1-carboxylic acid (endo-8-methyl-8-azabicyclo[3.2.1]oct-3-yl) ester

### (Compound 13)

Hydrochloride. M.p. > 260°C.

| Analysis | | | | |
|---|---|---|---|---|
| C₁₈H₂₁N₃O₄·HCl | Found % | C 56.69 | H 5.85 | N 11.03 |
| | Calc. % | C 56.91 | H 5.84 | N 11.06 |

### 7-methyl-2,3-dihydro 2 oxo-1H-benzimidazole-1-carboxylic acid (endo-8-methyl-8-azabicyclo[3.2.1]oct-3-yl) ester

### (Compound 14)

Hydrochloride. M.p. 250-251°C.

| Analysis | | | | |
|---|---|---|---|---|
| C₁₇H₂₁N₃O₃·HCl | Found % | C 57.58 | H 6.36 | N 11.73 |
| | Calc. % | C 58.03 | H 6.30 | N 11.94 |

### 5-trifluoromethyl-2,3-dihydro-2-oxo-1H-benzimidazole-1-carboxylic acid (endo-8-methyl-8-azabicyclo-[3.2.1]oct-3-yl) ester

### (Compound 15)

Hydrochloride. M.p. 222-224°C.

| Analysis | | | | |
|---|---|---|---|---|
| C₁₇H₁₈F₃N₃O₃·HCl | Found % | C 49.87 | H 4.75 | N 10.23 |
| | Calc. % | C 50.31 | H 4.72 | N 10.36 |

### 6-methoxy-2,3-dihydro-2-oxo-1H-benzimidazole-1-carboxylic acid (endo-8-methyl-8-azabicyclo[3.2.1]oct-3-yl) ester

### (Compound 16)

Hydrochloride. M.p. > 260°C.

| Analysis | | | | |
|---|---|---|---|---|
| C₁₇H₂₁N₃O₄·HCl | Found % | C 54.97 | H 6.09 | N 11.21 |
| | Calc. % | C 55.51 | H 6.03 | N 11.42 |

### 2,3-dihydro-2-oxo-1H-benzimidazole-1-carboxylic acid (endo-8-phenylmethyl-8-azabicyclo[3.2.1]oct-3-yl) ester

### (Compound 17)

M.p. 212-214°C.

| Analysis | | | | |
|---|---|---|---|---|
| C₂₂H₂₃N₃O₃ | Found % | C 69.30 | H 6.12 | N 11.03 |
| | Calc. % | C 70.00 | H 6.14 | N 11.13 |

### 6-nitro-2-oxo-1H-benzimidazole-1-carboxylic acid (endo-8-methyl-8-azabicyclo [3.2.1]oct-3-yl) ester

### (Compound 18)

Hydrochloride. M.p. > 260°C.

| Analysis | | | | |
|---|---|---|---|---|
| C₁₆H₁₈N₄O₅·HCl | Found % | C 49.10 | H 4.98 | N 14.46 |
| | Calc. % | C 50.20 | H 5.00 | N 14.64 |

### Example 11

### (Compound 1)

To a solution of 2,3-dihydro-1H-benzimidazole-2-one (0.9 g) in dry dimethylformamide (15 ml) 80% sodium hydride (0.4 g) was added at room temperature under stirring. Stirring was continued until hydrogen evolution stopped. Then the reaction mixture was cooled to 5°C. Endo-8-methyl-8-azabicyclo[3.2.1]oct-3-yl chloroformate hydrochloride (1. 38 g) was added portionwise at 5°C. After 30 min the temperature was allowed to reach 25°C and stirring was continued for further 60 min. The reaction mixture was evaporated to dryness then was taken up in acidic water and the aqueous phase was washed with ethyl acetate. The phase was made basic and extracted with methylene chloride. Evaporation of the solvent left a raw material which was purified by flash cromatography technique (eluent CH₂Cl₂/MeOH/32% NH₄OH 90:10:1) on Silcagel. 0.3 g. M.p. 190-191°C.
MS (C.l.): 302 m/e [M + H]⁺
IR (cm⁻¹) nujol: 1760, 1720

| Analysis | | | | |
|---|---|---|---|---|
| C₁₆H₁₉N₃O₃ | Found % | C 63.19 | H 6.44 | N 13.68 |
| | Calc. % | C 63.77 | H 6.36 | N 13.95 |

Analogously were obtained:

### 2,3-dihydro-2-oxo-1H-benzimidazole-1-carboxylic acid (endo-9-methyl-9-azabicyclo[3.3.1]non-3-yl) ester

### (Compound 19)

Citrate (freeze-dried). M.p. 96-100°C.
MS (C.l.): 316 m/e [M + H]⁺

| Analysis | | | | |
|---|---|---|---|---|
| C₁₇H₂₁N₃O₃·C₆H₈O₇ | Found % | C 51.36 | H 5.91 | N 7.74 |
| | Calc. % | C 54.43 | H 5.76 | N 8.28 |

### 2,3-dihydro-2-oxo-1H-benzimidazole-1-carboxylic acid (exo-9-methyl-9-azabicyclo[3.3.1]non-3-yl) ester

### (Compound 20)

Citrate. M.p. 77-80°C.
MS (C.l.): 316 m/e [M + H]⁺

| Analysis | | | | |
|---|---|---|---|---|
| C₁₇H₂₁N₃O₃·C₆H₈O₇ | Found % | C 51.09 | H 5.86 | N 7.97 |
| | Calc. % | C 54.43 | H 5.76 | N 8.28 |

### Example 12

### (Compound 1)

A solution of N-(2-aminophenyl)(endo-8-methyl-8-azabicyclo[3.2.1]oct-3-yl)carbamic acid ester (1.0 g) and carbonyldiimidazole (1.18 g) in benzene was heated to reflux for 1 hr. After cooling acidic water was added and the organic phase was discarded. The aqueous phase was made basic and extracted with methylene chloride. The organic phase was thoroughly washed with saturated NaCl solution, then dried over MgSO₄ and concentrated to dryness. Crystallization of the crude material from acetonitrile afforded the pure title compound (0.6 g).
M.p. 191-192°C.

| Analysis | | | | |
|---|---|---|---|---|
| C₁₆H₁₉N₃O₃ | Found % | C 63.66 | H 6.38 | N 13.89 |
| | Calc. % | C 63.77 | H 6.36 | N 13.95 |

### Example 13

### (Compound 1)

2,3-dihydro-2-oxo-1H-benzimidazole-1-carbonyl-chloride (2.15 g) was closely mixed with endo-8-methyl-8-azabicyclo[3.2.1]octan-3-ol (1.55 g) and the mixture was melted and let for 10 minutes at that temperature.

After the residue was taken up in acidic water and washed with ethyl-acetate. The aqueous phase was made strongly basic and again extracted.

The latter extracts were dried and evaporation of the solvent left the raw title compound which was crystallized from acetonitrile. 0.4 g.
M.p. 190-192°C.

| Analysis | | | | |
|---|---|---|---|---|
| C₁₆H₁₉N₃O₃ | Found % | C 63.45 | H 6.41 | N 13.81 |
| | Calc. % | C 63.77 | H 6.36 | N 13.95 |

Analogously were prepared:

### 2,3-dihydro-2-oxo-1H-benzimidazole-1-carboxylic acid (1-azabicyclo [2.2.2]oct-4-yl) ester

### (Compound 21)

Hydrochloride. M.p. 254-256°C.

| Analysis | | | | |
|---|---|---|---|---|
| C₁₅H₁₇N₃O₃·HCl | Found % | C 54.96 | H 5.71 | N 12.75 |
| | Calc. % | C 55.64 | H 5.60 | N 12.98 |

### 2,3-dihydro-2-oxo-1H-benzimidazole-1-carboxylic acid (endo-7-methyl-7-azabicyclo[2.2.1]ept-2-yl) ester

### (Compound 22)

Base. M.p. 175-178°C.

| Analysis | | | | |
|---|---|---|---|---|
| C₁₅H₁₇N₃O₃ | Found % | C 62.56 | H 5.96 | N 14.69 |
| | Calc. % | C 62.71 | H 5.96 | N 14.63 |

### 2,3-dihydro-2-oxo-1H-benzimidazole-1-carboxylic acid (exo-2-methyl-2-azabicyclo[2.2.2]oct-5-yl) ester

### (Compound 23)

Hydrochloride. M.p. 208-211°C.

| Analysis | | | | |
|---|---|---|---|---|
| C₁₆H₁₉N₃O₃·HCl | Found % | C 56.88 | H 6.12 | N 12.25 |
| | Calc. % | C 56.89 | H 5.97 | N 12.43 |

### 2,3-dihydro-2-oxo-1H-benzimidazole-1-carboxylic acid (endo-2-methyl-2-azabicyclo[2.2.2]oct-5-yl) ester

### (Compound 24)

Citrate M.p. 73-75°C.

| Analysis | | | | |
|---|---|---|---|---|
| C₁₆H₁₉N₃O₃·C₆H₈O₇ | Found % | C 52.96 | H 5.64 | N 8.39 |
| | Calc. % | C 53.55 | H 5.52 | N 8.52 |

### Example 14

### 3-methyl-2,3-dihydro-2-oxo-1H-benzimidazole-1-carboxylic acid (endo-8-methyl-8-azabicyclo[3.2.1]oct-3-yl) ester

### (Compound 25)

80% sodium hydride (0.04 g) was added portionwise to a solution of 2,3-dihydro-2-oxo-1H-benzimidazole-1-carboxylic acid (endo-8-methyl-8-azabicyclo[3.2.1]oct-3-yl) ester (0.4 g) in dry DMF (10 ml). After hydrogen evolution had subsided methyl iodide (0.082 ml) was added and the reaction mixture was stirred at room temperature for 2 hrs. The solvent was removed under vacuum and the residue was taken up in methylenechloride and washed with water. The organic phase was dried over MgSO₄ and concentrated to dryness. Pure title compound was obtained by flash chromatography technique (eluent: methylene chloride/methanol/32% NH₄OH 90:10:1) on Silicagel. The oily base was transformed into the hydrochloride salt. 0.21 g. M.p. > 250°C.
MS (C.l.): 352 m/e [M + H]⁺

| Analysis | | | | |
|---|---|---|---|---|
| C₁₇H₂₁N₃O₃·HCl | Found % | C 57.91 | H 6.34 | N 11.91 |
| | Calc. % | C 58.04 | H 6.30 | N 11.94 |

### Example 15

### (Compound 25)

A suspension of 3-methyl-2,3-dihydro-1H-benzimidazole-2-one (1.5 g) and trichloromethylchloroformate (2.43 ml) in dry o-dichlorobenzene (150 ml) was stirred overnight at 80°C. After cooling to 10°C the reactive intermediate was afforded by filtration. This compound was added to a solution of endo-8-methyl-8-azabicyclo[3.2.1]octan-3-ol (1.41 g) in dry pyridine (20 ml) at room temperature under stirring and, after the addition was over, the reaction mixture was stirred for 2 hrs at 80°C. After evaporation of the solvent the usual work-up afforded 0.7 g of the pure title compound as hydrochloride salt.
M.p. > 250°C.
MS (C.l.): 352 m/e [M + H]⁺

| Analysis | | | | |
|---|---|---|---|---|
| C₁₇H₂₁N₃O₃·HCl | Found % | C 57.85 | H 6.36 | N 11.83 |
| | Calc. % | C 58.04 | H 6.30 | N 11.94 |

### Example 16

### N-(endo-8-methyl-8-azabicyclo[3.2.1]oct-3-yl)-2,3-dihydro-2-oxo-1H-benzimidazole-1-carboxamide

### (Compound 26)

2,3-Dihydro-2-oxo-1H-benzimidazole-1-carbonyl chloride (1.5 g) was dissolved in tetrahydrofurane (40 ml) and to that solution a solution of endo-8-methyl-8-azabicyclo[3.2.1]octan-3-amine, dissolved in tetrahydrofurane (5 ml), was added dropwise at room temperature. When the addition was over a solid separated and the reaction mixture was stirred for 30 minutes, concentrated to dryness and taken up in diluted HCl.

The aqueous phase was washed with ethyl acetate, made basic with a saturated sodium carbonate and again extracted. The latter organic layers were concentrated to dryness giving 0.7 g of the crude product.

After crystallization from acetonitrile 0.17 g of the pure product were obtained. M.p. 205-207°C.
MS (C.l.): 301 m/e [M + H]⁺
IR (cm⁻¹): 1730, 1690

| Analysis | | | | |
|---|---|---|---|---|
| C₁₆H₂₀N₄O₂ | Found % | C 62.83 | H 6.75 | N 18.01 |
| | Calc. % | C 63.98 | H 6.71 | N 18.65 |

Similarly were prepared:

### N-(endo-9-methyl-9-azabicyclo[3.3.1]non-3-yl)-2,3-dihydro-2-oxo-1H-benzimidazole-1-carboxamide

### (Compound 27)

Hydrochloride. M.p. 269-270°C.
MS (0.1.): 315 m/e [M + H]⁺

| Analysis | | | | |
|---|---|---|---|---|
| C₁₇H₂₂N₄O₂·HCl | Found % | C 58.40 | H 6.62 | N 15.91 |
| | Calc. % | C 58.19 | H 6.61 | N 15.97 |

### N-(1-azabicyclo[2.2.2]oct-3-yl)-2,3-dihydro-2-oxo-1H-benzimidazole-1-carboxamide

### (Compound 28)

M.p. 196-198°C.
MS (C.l.): 287 m/e [M + H]⁺

| Analysis | | | | |
|---|---|---|---|---|
| C₁₅H₁₈N₄O₂ | Found % | C 62.34 | H 6.32 | N 19.34 |
| | Calc. % | C 62.92 | H 6.34 | N 19.57 |

### N-(endo-1-azabicyclo[3.3.1]non-4-yl)-2,3-dihydro-2-oxo-1H-benzimidazole-1-carboxamide

### (Compound 29)

M.p. 245-248°C.
MS (C.l.): 301 m/e [M + H]⁺

| Analysis | | | | |
|---|---|---|---|---|
| C₁₆H₂₀N₄O₂ | Found % | C 64.18 | H 6.80 | N 18.58 |
| | Calc. % | C 63.98 | H 6.71 | N 18.65 |

### N-(1-methylpiperidin-4-yl)-2,3-dihydro-2-oxo-1H-benzimidazole-1-carboxamide

### (Compound 30)

M.p. 194-197°C.
MS (C.l.): 275 m/e [M + H]⁺

| Analysis | | | | |
|---|---|---|---|---|
| C₁₄H₁₈N₄O₂ | Found % | C 61.18 | H 6.80 | N 20.34 |
| | Calc. % | C 61.30 | H 6.61 | N 20.42 |

### N-(endo-9-methyl-9-azabicyclo[3.3.1]non-3-yl)-3-methyl-2,3-dihydro-2-oxo-1H-benzimidazole-1-carboxamide

### (Compound 31)

M.p. 175-176°C.
MS (C.l.): 329 m/e [M + H]⁺

| Analysis | | | | |
|---|---|---|---|---|
| C₁₈H₂₄N₄O₂ | Found % | C 65.39 | H 7.32 | N 16.92 |
| | Calc. % | C 65.83 | H 7.36 | N 17.06 |

### N-(endo-8-methyl-8-azabicyclo[3.2.1]oct-3-yl)-3-methyl-2,3-dihydro-2-oxo-1H-benzimidazole-1-carboxamide

### (Compound 32)

Hydrochloride. M.p. 269-270°C
MS (C.l.): 315 m/e [M + H]⁺

| Analysis | | | | |
|---|---|---|---|---|
| C₁₇H₂₂N₄O₂·HCl | Found % | C 58.14 | H 6.49 | N 16.01 |
| | Calc. % | C 58.19 | H 6.61 | N 15.97 |

### N-methyl-N-(endo-9-methyl-9-azabicyclo[3.3.1]non-3-yl)-2,3-dihydro-2-oxo-1H-benzimidazole-1-carboxamide

### (Compound 33)

M.p. 198-200°C.
MS (C.l.): 329 m/e [M + H]⁺

| Analysis | | | | |
|---|---|---|---|---|
| C₁₈H₂₄N₄O₂ | Found % | C 65.72 | H 7.53 | N 16.85 |
| | Calc. % | C 65.83 | H 7.37 | N 17.06 |

### N-(endo-9-methyl-9-azabicyclo[3.3.1]non-3-yl)-N-[(2,4-dimethoxyphenyl) methyl]-2,3-dihydro-2-oxo-1H-benzimidazole-1-carboxamide

### (Compound 34)

M.p. 100-104°C.
MS (0.1.): 465 m/e [M + H]⁺

| Analysis | | | | |
|---|---|---|---|---|
| C₂₆H₃₂N₄O₄ | Found % | C 66.31 | H 6.89 | N 12.31 |
| | Calc. % | C 67.20 | H 6.95 | N 12.07 |

### N-(endo-8-phenylmethyl-8-azabicyclo[3.2.1]oct-3-yl)-2,3-dihydro-2-oxo-1H-benzimidazole-1-carboxamide

### (Compound 35)

M.p. 221-224°C

| Analysis | | | | |
|---|---|---|---|---|
| C₂₂H₂₄N₄O₂ | Found % | C 70.02 | H 6.41 | N 14.69 |
| | Calc. % | C 70.19 | H 6.43 | N 14.88 |

### Example 17

### N-(endo-9-methyl-9-azabicyclo[3.3.1]non-3-yl)-2,3-dihydro-2-oxo-1H-benzimidazole-1-carboxamide

### (Compound 27)

A solution of N-(endo-9-methyl-9-azabicyclo[3.3.1]non-3-yl)-N-[(2,4-dimethoxy phenyl)methyl]-2,3-dihydro-2-oxo-1H-benzimidazole-1-carboxamide (1.0 g) and anisole (0.6 g)in trifluoroacetic acid (10 ml) was stirred at room temperature for 12 hrs. The reaction mixture was then concentrated to dryness and the residue oil was purified by flash-chromatography on silica-gel: eluent methylene chloride-methanol-32% ammonium hydroxide 80:20:2. 0.12 g of the title compound were obtained.
M.p. 180-182°C.

| Analysis | | | | |
|---|---|---|---|---|
| C₁₇H₂₂N₄O₂ | Found % | C 64.83 | H 7.02 | N 17.75 |
| | Calc. % | C 64.95 | H 7.05 | N 17.82 |

Analogously and starting from the appropriate precursor was also obtained:

### N-(endo-9-methyl-9-azabicyclo[3.3.1]non-3-yl)3-methyl-2,3-dihydro-2-oxo-1H-benzimidazole-1-carboxamide

### (Compound 31)

M.p. 175-176°C.

| Analysis | | | | |
|---|---|---|---|---|
| C₁₈H₂₄N₄O₂ | Found % | C 65.12 | H 7.38 | N 16.94 |
| | Calc. % | C 65.83 | H 7.36 | N 17.06 |

### Example 18

### 6-acetyl amino-2,3-dihydro-2-oxo-1H-benzimidazole-1-carboxylic acid (endo-8-methyl-8-azabicyclo[3.2.1]oct-3-yl) ester

### (Compound 36)

a) Sodium hypophosphite (2.37 g) was added portiowise and under stirring to a suspension of 6-nitro-2,3-dihydro-2-oxo-1H-benzimidazole-1-carboxylic acid (endo-8-methyl-8-azabicyclo[3.2.1]oct-3-yl)ester, hydrochloride (2.85 g) and 10% Pd/C (0.28 g) in 80 ml of water. When the addition was over, the reaction mixture was heated to the boiling temperature for 30 minutes. After cooling it was filtered, made basic with saturated sodium carbonate and extracted with methylene chloride.
The organic layer, after drying over MgSO₄ and evaporation of the solvent, gave 0.88 g of the crude product. By adding alcoholic chloridric acid 0.6 g of 6-amino-2,3-dihydro-2-oxo-1H-benzimidazole-1-carboxylic acid (endo-8-methyl-8-azabicyclo[3.2.1]oct-3-yl) ester, dihydrochloride were obtained by crystallization. M.p. > 260°C.
b) Pyridine (1.2 ml) and acetic anhydride (0.14 ml) were added to a solution of 6-amino-2,3-dihydro-2-oxo-1H-benzimidazole-1-carboxylic acid (endo-8-methyl-8-azabicyclo[3.2.1]oct-3-yl) ester (0.48 g) in tetrahydrofurane (10 ml). The resulting mixture was stirred at room temperature for 30 minutes, concentrated to dryness and the residue was taken up in water. It was made basic and from the mother liquors the title product slowly crystallized. Its hydrochloride was then obtained in conventional manner. Yield 0.3 g. M.p. > 260°C.
MS (0.1.): 359 m/e [M + H]⁺

| Analysis | | | | |
|---|---|---|---|---|
| C₁₈H₂₂N₄O₄·HCl | Found % | C 54.02 | H 5.88 | N 13.51 |
| | Calc. % | C 54.75 | H 5.62 | N 14.19 |

### Example 19

### 2,3-dihydro-2-oxo-1H-benzimidazole-1-carboxylic acid (endo-8-methyl-8-azabicyclo[3.2.1]oct-3-yl)ester, methobromide

### (Compound 37)

A solution of 2,3-dihydro-2-oxo-1H-benzimidazole-1-carboxylic acid (endo-8-methyl-8-azabicyclo[3.2.1]oct-3-yl) ester (0.5 g) in acetone (60 ml) was added in 40 minutes to a mixture of acetone (20 ml) and methylbromide [2M solution in diethyl ether (20 ml)], cooled at 5°C. The resulting mixture was left overnight at room temperature. The crude product separated as a solid and was recovered by filtration. After crystallization from ethanol 0.2 g of the pure product were obtained. M.p. > 260°C.

| Analysis | | | | |
|---|---|---|---|---|
| C₁₇H₂₂BrN₃O₃ | Found % | C 51.02 | H 5.65 | N 10.33 |
| | Calc. % | C 51.48 | H 5.60 | N 10.60 |

### Example 20

### 2,3-dihydro-2-oxo-1H-benzimidazole-1-carboxylic acid (endo-8-azabicyclo [3.2.1]oct-3-yl) ester

### (Compound 38)

A suspension of 2,3-dihydro-2-oxo-1H-benzimidazole-1-carbonyl chloride (1.3 g) and endo-8-azabicyclo[3.2.1]octan-3-ol hydrochloride (1.0 g) in o-dichlorobenzene (5 ml) was heated at 180°C for 1 hour under stirring.

The reaction mixture was then allowed to cool and the solvent was removed by filtration. The crude product so obtained was washed with a little ethanol and crystallized from ethanol. 1.1 g of the desired product was obtained. M.p. > 260°C.
MS (C.l.): 288 m/e [M + H]⁺

| Analysis | | | | |
|---|---|---|---|---|
| C₁₅H₁₇N₃O₃·HCl | Found % | C 55.15 | H 5.61 | N 12.70 |
| | Calc. % | C 55.64 | H 5.60 | N 12.98 |

### Example 21

### N-(endo-8-azabicyclo[3.2.1]oct-3-yl)-2,3-dihydro-2-oxo-1H-benzimidazole-1-carboxamide

### (Compound 39)

A suspension of N-(endo-8-phenylmethyl-8-azabicyclo[3.2.1]oct-3-yl)-2,3-dihydro-2-oxo-1H-benzimidazole-1-carboxamide (1.0 g) in 1:1 aqueous ethanol (50 ml) was hydrogenated at room temperature and 10 atm. pressure in the presence of 10% Pd/C. After the usual workup 0.6 g of the title compound were obtained.
Hydrochloride. M.p. > 250°C.

| Analysis | | | | |
|---|---|---|---|---|
| C₁₅H₁₈N₄O₂·HCl | Found % | C 55.64 | H 5.96 | N 17.21 |
| | Calc. % | C 55.81 | H 5.93 | N 17.36 |

### Example 22

### 2,3-dihydro-2-oxo-1H-benzimidazole-1-carboxylic acid (endo-8-cyclopropylmethyl-8-azabicyclo[3.2.1]oct-3-yl) ester

### (Compound 40)

Anhydrous sodium carbonate (1.0 g) and cyclopropylmethyl bromide (0.3 g) were added to a solution of 2,3-dihydro-2-oxo-1H-benzimidazole-1-carboxylic acid (endo-8-azabicyclo[3.2.1]oct-3-yl) ester (0.5 g) in ethanol (20 ml). The resulting suspension was refluxed for 3 hours and, after cooling, the unsoluble product was removed by filtration. The mother liquors were then concentrated to dryness. The residue was taken up in water, made basic with sodium carbonate and extracted with ethyl acetate.

After drying over MgSO₄, 0.4 g of the crude product were obtained. Its hydrochloride was prepared in conventional manner. Yield 0.3 g.
M.p. > 270°C.
MS (C.l.): 378 m/e [M + H]⁺

| Analysis | | | | |
|---|---|---|---|---|
| C₁₉H₂₃N₃O₃·HCl | Found % | C 59.71 | H 6.42 | N 11.06 |
| | Calc. % | C 60.37 | H 6.40 | N 11.27 |

### Example 23

### 2,3-dihydro-2-oxo-1H-benzimidazole-1-carboxylic acid (endo-8-iminomethyl-8-azabicyclo[3.2.1]oct-3-yl), ester

### (Compound 41)

Ethyl formimidate hydrochloride (0.5 g) was added portionwise to a solution of 2,3-dihydro-2-oxo-1H-benzimidazole-1-carboxylic acid (endo-8-azabicyclo[3.2.1]oct-3-yl) ester (1.0 g) in ethanol (40 ml). The solution was stirred at room temperature for 1 hour, and the solid so separated was recovered by filtration. Yield 0.4 g. Hydrochloride.
M.p. 210-212°C.
MS (C.l.): 351 m/e [M + H]⁺

| Analysis | | | | |
|---|---|---|---|---|
| C₁₆H₁₈N₄O₃·HCl | Found % | C 53.96 | H 5.51 | N 15.62 |
| | Calc. % | C 54.78 | H 5.46 | N 15.97 |

Analogously was obtained:

### N-(endo-8-iminomethyl-8-azabicyclo[3.2.1]non-3-yl)-2,3-dihydro-2-oxo-1H-benzimidazole-1-carboxamide

### (Compound 42)

Hydrochloride (freeze-dried). M.p. 65-70°C
MS (C.l.): 350 m/e [M + H]⁺

| Analysis | | | | |
|---|---|---|---|---|
| C₁₆N₁₉N₅O₂·HCl | Found % | C 53.86 | H 5.84 | N 19.87 |
| | Calc. % | C 54.34 | H 5.76 | N 20.02 |

### Example 24

### 2,3-dihydro-2-oxo-1H-benzimidazole-1-carboxylic acid (endo-8-[1'-(methylimino)ethyl]-8-azabicyclo[3.2.1]oct-3-yl) ester

### (Compound 43)

Phenyl N-methylacetimidate (0.52 g) was added to a solution of 2,3-dihydro-2-oxo-1H-benzimidazole-1-carboxylic acid(endo-8-azabicyclo [3.2.1]oct-3-yl) ester, hydrochloride (1.0 g) in ethanol (20 ml). The reaction mixture was stirred for 3 hours at 60°C. After evaporation of the solvent the crude product was purified by flash-chromatograpy technique eluent: n-propanol-water-acetic acid 90:10:10. Yield 0.4 g.
Hydrochloride. Freeze-dried. M.p. 68-72°C.
MS (C.l.): 379 m/e [M + H]⁺

| Analysis | | | | |
|---|---|---|---|---|
| C₁₈H₂₂N₄O₃·HCl | Found % | C 56.83 | H 6.09 | N 14.91 |
| | Calc. % | C 57.07 | H 6.12 | N 14.79 |

### Example 25

### 2,3-dihydro-2-oxo-1H-benzimidazole-1-carboxylic acid (endo-8-amidino-8-azabicyclo[3.2.1]oct-3-yl) ester

### (Compound 44)

Cyanamide (0.26 g) was added under stirring to a suspension of 2,3-dihydro-2-oxo-1H-benzimidazole-1-carboxylic acid (endo-8-azabicyclo[3.2.1]oct-3-yl) ester, hydrochloride in 0.5 ml of water. The homogenized reaction mixture was heated to 130°C and kept under stirring at that temperature for 2 hours. After cooling the crude product was purified by flash-chromatography on silica-gel: eluent n-propanol-acetic acid-water 90:10:10.

After freeze-drying 0.3 g of the pure product were obtained.
M.p. 70-75°C.
Ms (C.l.) : 366 m/e [M + H]⁺

| Analysis | | | | |
|---|---|---|---|---|
| C₁₆H₁₉N₅O₃·HCl | Found % | C 51.73 | H 5.45 | N 19.17 |
| | Calc. % | C 52.53 | H 5.51 | N 19.14 |

The following examples of pharmaceutical compositions according to the invention are reported:

### Example 26

| Tablets | |
|---|---|
| - active ingredient | 250 mg |
| - lactose | 270 mg |
| - corn starch | 76 mg |
| - magnesium stearate | 4 mg |

Method of preparation: The active ingredient, lactose and corn starch were mixed and homogeneously moistened with water. After screening of the moist mass and drying in a tray drier, the mixture was again passed throught a screen and magnesium stearate was added. Then the mixture was pressed into tablets weighing 600 mg each. Each tablet contains 250 mg of active ingredient.

### Example 27

| Capsules | |
|---|---|
| - active ingredient | 250 mg |
| - lactose | 148 mg |
| - magnesium stearate | 2 mg |

Method of preparation: The active ingredient was mixed with auxiliary products, and the mixture was passed through a screen and mixed homogeneously in a suitable device. The resulting mixture was filled into hard gelatine capsules (400 mg per capsule); each capsule contains 250 mg of active ingredient.

### Example 28

| Ampoules | |
|---|---|
| - active ingredient | 50 mg |
| - Sodium chloride | 10 mg |

Method of preparation: The active ingredient and sodium chloride were dissolved in an appropriate amount of water for injection. The resulting solution was filtered anf filled into ampoules under sterile conditions. Each ampoule contains 50 mg of active ingredient.

### Example 29

| Suppositories | |
|---|---|
| - active ingredient | 250 mg |
| - semisynthetic glicerides of fatty acids | 950 mg |

Method of preparation: The semisynthetic glicerides of fatty acids were melted and the active ingredient was added while stirring homogeneously. After cooling at a proper temperature the mass was poured into preformed moulds for suppositories weighing 1200 mg each. Each suppository contains 250 mg of active ingredient.

### Example 30

| Oral drops | |
|---|---|
| - active ingredient | 50 mg |
| - sorbitol | 350 mg |
| - propylene glycol | 100 mg |
| - citric acid | 1 mg |
| - sodium citrate | 3 mg |
| - demineralized water q.s. | 1 ml |

Method of preparation: The active ingredient, citric acid and sodium citrate were dissolved in a mixture of a proper amount of water and propylene glycol. Then sorbitol was added and the final solution was filtered. The solution contains 5% of active ingredient and is administered by using a proper dropper.

## Claims

1. Compounds of general formula (I) 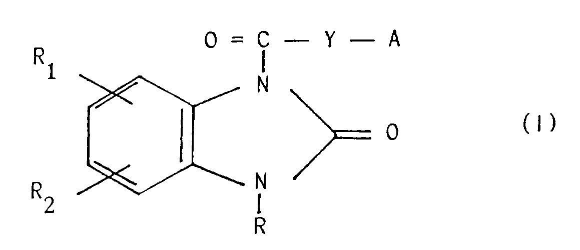 wherein R represents a hydrogen atom, C₁₋₆ alkyl, alkenyl with up to 6 carbon atoms and alkynyl with up to 6 carbon atoms; R₁ and R₂ may be at the same time or not a hydrogen atom, halogen, trifluoromethyl, C₁₋₆ alkyl, C₁₋₆ alkoxy, C₁₋₆ alkylthio, C₁₋₆ acyl, carboxyl, C₁₋₆ alkoxycarbonyl, hydroxy, nitro, amino optionally C₁₋₄ alkyl N-mono or di-substituted, C₁₋₆ acylamino, C₁₋₆ alkoxycarbonylamino, carbamoyl optionally C₁₋₄ alkyl N-mono or di-substituted, cyano, C₁₋₆alkylsulphinyl, C₁₋₆ alkylsulphonyl, amino sulphonyl optionally C₁₋₄ alkyl N-mono or disubstituted, C₁₋₄ alkyl N-mono or di-substituted aminosulphonylamino, aminosulphonylamino; Y is oxygen or is N - R₃ in which R₃ is a hydrogen, a C₁₋₆alkyl or benzyl optionally substituted by one or more C₁₋₆ alkoxy; A is a group selected from: 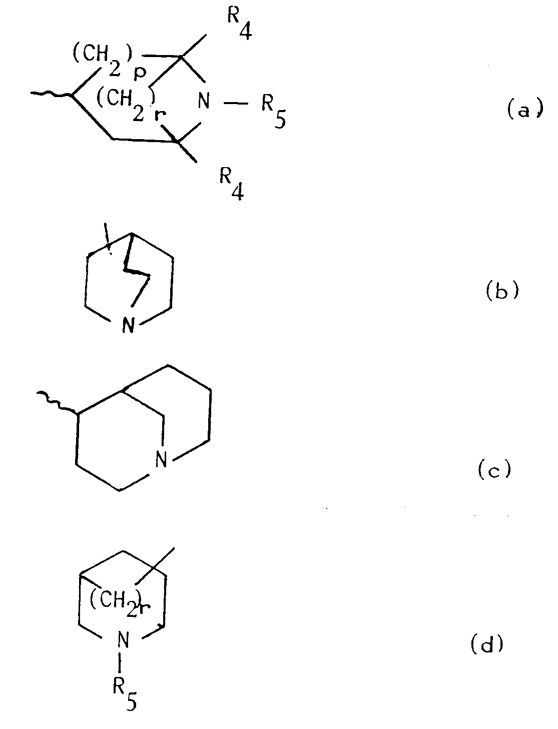 wherein p is 0, 1; r is 0, 1, 2, 3; R₄ is hydrogen atom or a C₁₋₄ alkyl; R₅ is a hydrogen atom, C₁₋₆ alkyl, C₃₋₈ cycloalkyl, C₃₋₈ cycloalkyl C₁₋₄ alkyl, C₁₋₄ alkyl substituted with phenyl or R₅ is a group of formula 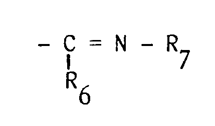 wherein R₆ is hydrogen atom, C₁₋₄ alkyl or an amino group and R₇ is hydrogen atom or C₁₋₆ alkyl, tautomers thereof and acid addition salts of the aforesaid compounds.

2. Physiologically compatible acid addition salts of compounds of general formula (I) according to claim 1.

3. Salts according to claim 2, characterized in that the physiologically compatible acids are hydrochloric, hydrobromic, sulphuric, acetic, citric and tartaric acid.

4. Compounds of general formula (I) according to claim 1, characterized in that A represents endo-8-methyl-8-azabicyclo-[3.2.1]oct-3-yl, R₁ and R₂ are H, R is H or CH₃ and Y is oxygen or a NH group, tautomers thereof and acid addition salts of the aforesaid compounds.

5. Compounds of general formula (I) according to claim 1, characterized in that A represents endo-9-methyl-9-azabicyclo[3.3.1]non-3-yl, R₁ and R₂ are H, R is H or CH₃ and Y is oxygen or a NH group, tautomers thereof and acid addition salts of the aforesaid compounds.

6. Compounds of general formula (I) according to claim 1, characterized in that A represents endo-1-azabicyclo[3.3.1]non-4-yl, R₁ and R₂ are H, R is H or CH₃ and Y is oxygen or a NH group, tautomers thereof and acid addition salts of the aforesaid compounds.

7. Compounds of general formula (I) according to claim 1, characterized in that A is 1-azabicyclo[2.2.2]oct-3-yl, R₁ and R₂ are H, R is H or CH₃ and Y is oxygen or a NH group, tautomers thereof and acid addition salts of the aforesaid compounds.

8. Physiologically compatible acid addition salts of compounds of general formula (I) according to claims 4, 5, 6 or 7.

9. Salts according to claim 8, characterized in that the physiologically compatible acids are hydrochloric, hydrobromic, sulphuric, acetic, citric and tartaric acid.

10. A compound according to claim 1 which is N-(1-azabicyclo[2.2.2]-oct-3-yl)-2,3-dihydro-2-oxo-1H-benzimidazole-1-carboxamide.

11. A compound according to claim 1 which is N-(endo-9-methyl-9-azabicyclo[3.3.1]non-3-yl)-3-methyl-2,3-dihydro-2-oxo-1H-benzimidazole-1-carboxamide.

12. A compound according to claim 1 which is N-(endo-8-methyl-8-azabicyclo[3.2.1]oct-3-yl)-2,3-dihydro-2-oxo-1H-benzimidazole-1-carboxamide.

13. A compound according to claim 1 which is N-(endo-9-methyl-9-azabicyclo[3.3.1]non-3-yl)-2,3-dihydro-2-oxo-1H-benzimidazole-1-carboxamide

14. Physiologically compatible acid addition salts of compounds according to claim 10, 11, 12 or 13.

15. Salts according to claim 14, characterized in that the physiologically compatible acids are hydrochloric, hydrobromic, sulphuric, acetic, citric and tartaric acid.

16. Process for the preparation of compounds of general formula (I) according to claim 1, in which R is H, characterized in that a compound of general formula (II) 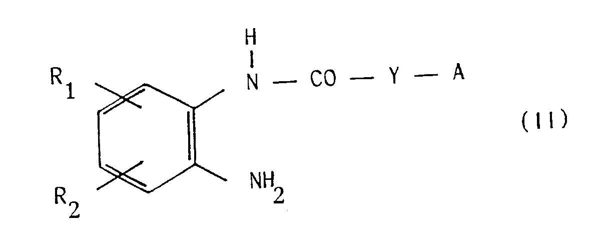 in which R₁, R₂, Y and A are as defined in claim 1, is reacted with a reactive carbonyl derivative of general formula (III) 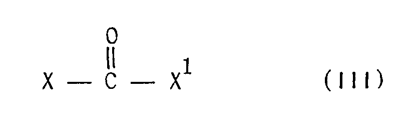 in which X and X¹ are leaving groups, identical or different from each other, in aprotic solvent at a temperature ranging from 0° to 100°C.

17. Process according to claim 16, characterized in that the leaving group is selected from chlorine, trichloromethoxy, methoxy, ethoxy or imidazolyl.

18. Process for the preparation of compounds of general formula (I) according to claim 1, in which R₁ and R₂ are both hydrogen atoms, characterized in that a compound of formula (VII) 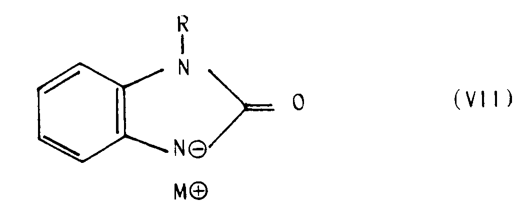 in which M is a metal atom, is reacted with a reactive intermediate of general formula (VI) 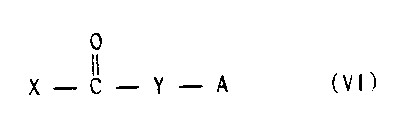 in which Y and A are as defined in claim 1 and X in claim 16, in a polar aprotic solvent at a temperature ranging from 0° to 100°C.

19. Process according to claim 18, characterized in that the metal atom is selected from sodium, potassium and lithium.

20. Process for the preparation of compounds of general formula (I) according to claim 1, in which R₁ and R₂ are both hydrogen atoms, characterized in that a reactive derivative of formula (VIII) 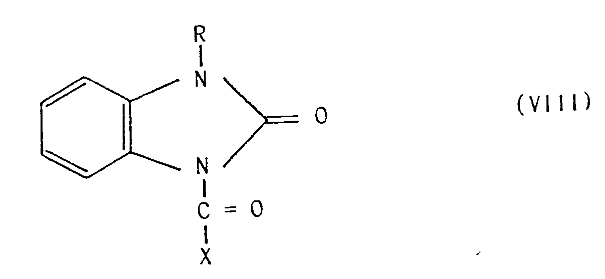 in which R is as defined in claim 1 and X in claim 16 is reacted with a compound of formula (IX)
Z ― Y ― A (IX)
in which Z represents a hydrogen atom, lithium, sodium or potassium, Y and A are as defined in claim 1, in an aprotic solvent in the presence of an acid acceptor at a temperature ranging from 0° to 200°C.

21. Pharmaceutical compositions comprising as active ingredient at least one compound of general formula (I) according to claim 1 or tautomer or physiologically compatible acid addition salts thereof in association with pharmaceutical carriers or excipients.

22. Pharmaceutical compositions according to claim 21 as 5-HT agents.

23. Pharmaceutical compositions according to claim 21 for the use in the treatment of patients suffering from chemotherapy and radiation induced nausea and emesis or from delayed gastric empting, in the gastrointestinal motility disorders and in particular in dyspepsia, flatulence, oesophageal reflux, irritable bowel syndrome and hypokinesia.

24. Pharmaceutical compositions according to claim 21 for the use in the treatment of patients suffering from motion sikness, migraine, cluster headhaches, anxiety and psychosis.

## Patentansprüche

1. Verbindungen der allgemeinen Formel (I) 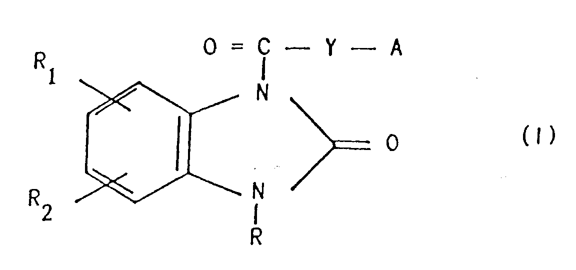 worin R für ein Wasserstoffatom, C₁₋₆-Alkyl, Alkenyl mit bis zu 6 Kohlenstoffatomen und Alkinyl mit bis zu 6 Kohlenstoffatomen steht; R₁ und R₂ gegebenenfalls gleichzeitig für ein Wasserstoffatom, ein Halogenatom, Trifluormethyl, C₁₋₆-Alkyl, C₁₋₆-Alkoxy, C₁₋₆-Alkylthio, C₁₋₆-Acyl, Carboxyl, C₁₋₆-Alkoxycarbonyl, Hydroxy, Nitro, gegebenenfalls mit C₁₋₄-Alkyl N-mono- oder di-substituiertes Amino, C₁₋₆-Acylamino, C₁₋₆-Alkoxycarbonylamino, gegebenenfalls mit C₁₋₄-Alkyl N-mono- oder di-substituiertes Carbamoyl, Cyano, C₁₋₆-Alkylsulfinyl, C₁₋₆-Alkylsulfonyl, gegebenenfalls mit C₁₋₄-Alkyl N-mono- oder di-substituiertes Aminosulfonyl, mit C₁₋₄-Alkyl N-mono- oder di-substituiertes Aminosulfonylamino oder Aminosulfonylamino stehen; Y für ein Sauerstoffatom oder für N-R₃ steht, worin R₃ ein Wasserstoffatom bedeutet oder für einen C₁₋₆-Alkyl- oder Benzylrest steht, der gegebenenfalls durch eine oder mehrere C₁₋₆-Alkoxygruppen substituiert ist; A für eine Gruppe steht, die ausgewählt ist unter: 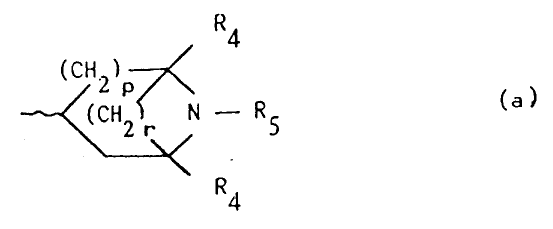 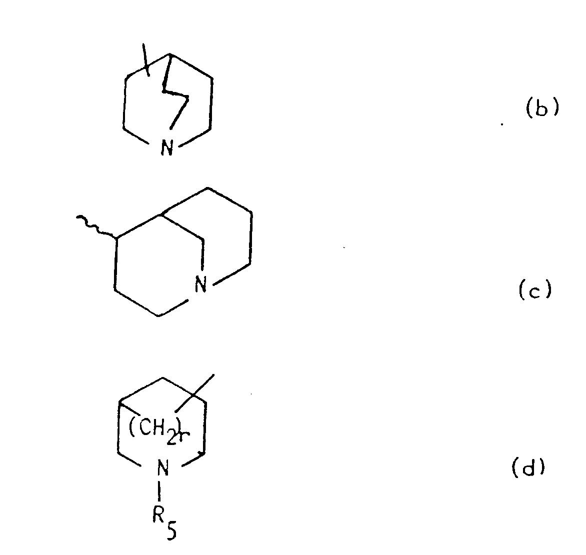 worin p für 0 oder 1 steht; r für 0, 1, 2 oder 3 steht; R₄ für ein Wasserstoffatom oder eine C₁₋₄-Alkylgruppe steht; R₅ für ein Wasserstoffatom, eine C₁₋₆-Alkyl-, C₃₋₈-Cycloalkyl-, C₃₋₈-Cycloalkyl-C₁₋₄-alkylgruppe, für eine gegebenenfalls durch Phenyl substituierte C₁₋₄-Alkylgruppe steht, oder R₅ eine Gruppe der Formel 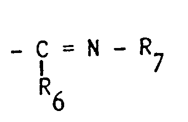 bedeutet, worin R₆ ein Wasserstoffatom,
eine C₁₋₄-Alkyl- oder eine Aminogruppe bedeutet, und R₇ ein Wasserstoffatom oder eine C₁₋₆-Alkylgruppe bedeutet,
sowie die tautomeren Formen und die Säureadditionssalze der oben genannten Verbindungen.

2. Physiologisch verträgliche Säureadditionssalze von Verbindungen der allgemeinen Formel (I) gemäß Anspruch 1.

3. Salze gemäß Anspruch 2, dadurch gekennzeichnet, daß die physiologisch verträglichen Säuren ausgewählt sind unter Chlorwasserstoff-, Bromwasserstoff-, Schwefel-, Essig-, Citronen-, und Weinsäure.

4. Verbindungen der allgemeinen Formel (I) gemäß Anspruch 1, dadurch gekennzeichnet, daß A für Endo-8-methyl-8-azabicyclo-[3.2.1]oct-3-yl steht, R₁ und R₂ für H stehen, R für H oder CH₃ steht und Y für ein Sauerstoffatom oder eine NH-Gruppe steht, sowie die tautomeren Formen und Säureadditionssalze der oben erwähnten Verbindungen.

5. Verbindungen der allgemeinen Formel (I) gemäß Anspruch 1, dadurch gekennzeichnet, daß A für Endo-9-methyl-9-azabicyclo-[3.3.1]non-3-yl steht, R₁ und R₂ für H stehen, R für H oder CH₃ steht und Y für ein Sauerstoffatom oder eine NH-Gruppe steht, sowie die tautomeren Formen und Säureadditionssalze der oben erwähnten Verbindungen.

6. Verbindungen der allgemeinen Formel (I) gemäß Anspruch 1, dadurch gekennzeichnet, daß A für Endo-1-azabicyclo[3.3.1]non-4-yl steht, R₁ und R₂ für H stehen, R für H oder CH₃ steht und Y für ein Sauerstoffatom oder eine NH-Gruppe steht, sowie die tautomeren Formen und die Säureadditionssalze der oben erwähnten Verbindungen.

7. Verbindungen der allgemeinen Formel (I) gemäß Anspruch 1, dadurch gekennzeichnet, daß A für 1-Azabicyclo[2.2.2]oct-3-yl steht, R₁ und R₂ für H stehen, R für H oder CH₃ steht und Y für ein Sauerstoffatom oder eine NH-Gruppe steht, sowie die tautomeren Formen und Säureadditionssalze der oben erwähnten Verbindungen.

8. Physiologisch verträgliche Säureadditionssalze von Verbindungen der allgemeinen Formel (I) gemäß den Ansprüchen 4, 5, 6 oder 7.

9. Salze gemäß Anspruch 8, dadurch gekennzeichnet, daß die physiologisch verträglichen Säuren ausgewählt sind unter Chlorwasserstoff-, Bromwasserstoff-, Schwefel-, Essig-, Citronen- und Weinsäure.

10. Verbindung nach Anspruch 1, nämlich N-(1-Azabicyclo[2.2.2]oct-3-yl)-2,3-dihydro-2-oxo-1H-benzimidazol-1-carboxamid.

11. Verbindung nach Anspruch 1, nämlich N-(Endo-9-methyl-9-azabicyclo[3.3.1]non-3-yl)-3-methyl-2,3-dihydro-2-oxo-1H-benzimidazol-1-carboxamid.

12. Verbindung nach Anspruch 1, nämlich N-(Endo-8-methyl-8-azabicyclo[3.2.1]oct-3-yl)-2,3-dihydro-2-oxo-1H-benzimidazol-1-carboxamid.

13. Verbindung nach Anspruch 1, nämlich N-(Endo-9-methyl-9-azabicyclo[3.3.1]non-3-yl)-2,3-dihydro-2-oxo-1H-benzimidazol-1-carboxamid.

14. Physiologisch verträgliche Säureadditionssalze von Verbindungen gemäß den Ansprüchen 10, 11, 12 oder 13.

15. Salze gemäß Anspruch 14, dadurch gekennzeichnet, daß die physiologisch verträglichen Säuren ausgewählt sind unter Chlorwasserstoff-, Bromwasserstoff-, Schwefel-, Essig-, Citronen- und Weinsäure.

16. Verfahren zur Herstellung von Verbindungen der allgemeinen Formel (I) gemäß Anspruch 1, worin R für H steht, dadurch gekennzeichnet, daß eine Verbindung der allgemeinen Formel (II) 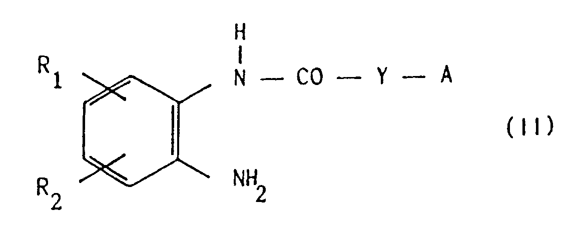 worin R₁, R₂, Y und A die in Anspruch 1 angegebenen Bedeutungen besitzen, mit einem reaktiven Carbonylderivat der allgemeinen Formel (III) 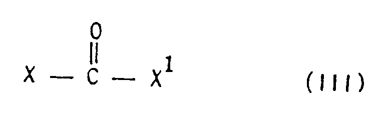 worin X und X¹ gleiche oder voneinander verschiedene Abgangsgruppen sind, in einem aprotischen Lösungsmittel bei einer Temperatur im Bereich von 0°C bis 100°C umgesetzt wird.

17. Verfahren nach Anspruch 16, dadurch gekennzeichnet, daß die Abgangsgruppe ausgewählt ist unter Chlor, Trichlormethoxy, Methoxy, Ethoxy oder Imidazolyl.

18. Verfahren zur Herstellung von Verbindungen der allgemeinen Formel (I) gemäß Anspruch 1, worin R₁ und R₂ jeweils ein Wasserstoffatom bedeuten, dadurch gekennzeichnet, daß man eine Verbindung der Formel (VII) 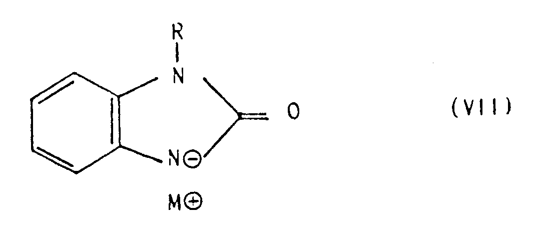 worin M für ein Metallatom steht, mit einem reaktiven Zwischenprodukt der allgemeinen Formel (VI) 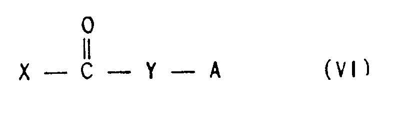 worin Y und A die in Anspruch 1 angegebenen Bedeutungen besitzen und X die in Anspruch 16 angegebenen Bedeutungen besitzt, in einem polaren, aprotischen Lösungsmittel bei einer Temperatur im Bereich von 0°C bis 100°C umsetzt.

19. Verfahren nach Anspruch 18, dadurch gekennzeichnet, daß das Metallatom ausgewählt ist unter Natrium, Kalium und Lithium.

20. Verfahren zur Herstellung von Verbindungen der allgemeinen Formel (I) gemäß Anspruch 1, worin R₁ und R₂ jeweils ein Wasserstoffatom bedeuten, dadurch gekennzeichnet, daß man ein reaktives Derivat der Formel (VIII) 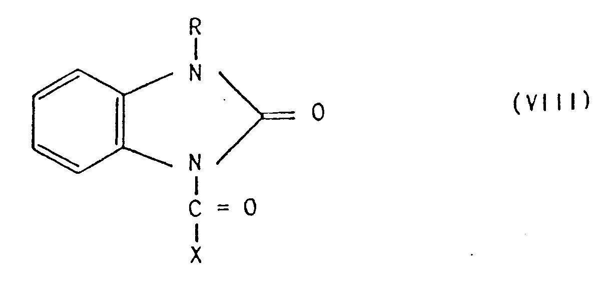 worin R wie in Anspruch 1 und X wie in Anspruch 16 definiert ist, mit einer Verbindung der Formel (IX)
Z-Y-A (IX)
worin Z für ein Wasserstoffatom, Lithium, Natrium oder Kalium steht, Y und A die in Anspruch 1 angegebenen Bedeutungen besitzen, in einem aprotischen Lösungsmittel in Gegenwart eines Säureakzeptors bei einer Temperatur im Bereich von 0°C bis 200°C umsetzt.

21. Pharmazeutische Mittel, umfassend als aktiven Bestandteil wenigstens eine Verbindung der allgemeinen Formel (I) gemäß Anspruch 1 oder eine tautomere Form oder ein physiologisch verträgliches Säureadditionssalz davon in Kombination mit pharmazeutischen Trägern oder Exzipienten.

22. Pharmazeutische Mittel gemäß Anspruch 21 als 5-HT-Agenzien.

23. Pharmazeutische Mittel gemäß Anspruch 21 zur Verwendung bei der Behandlung von Patienten, die an Chemotherapie- und Bestrahlungs-induziertem Brechreiz und Erbrechen oder an verzögerter Magenentleerung, an Störungen der gastrointestinalen Motilität und insbesondere an Dyspepsie, Flatulenz, Ösophagus-Rückfluß, reizbarem Darmsyndrom und Hypokinese leiden.

24. Pharmazeutische Mittel gemäß Anspruch 21 zur Verwendung bei der Behandlung von Patienten, die an Kinetose, Migräne, einseitigem Kopfschmerz, Angst und/oder Psychose leiden.

## Revendications

1. Composés de formule générale (I) 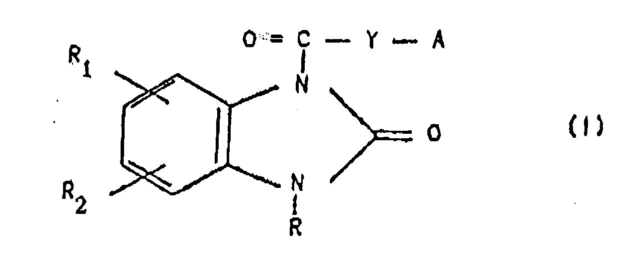 dans laquelle R représente un atome d'hydrogène, un alkyle en C₁₋₆, un alcényle ayant jusqu'à 6 atomes de carbone et un alcynyle ayant jusqu'à 6 atomes de carbone; R₁ et R₂ peuvent être simultanément ou non un atome d'hydrogène, un halogène, un groupe trifluorométhyle, alkyle en C₁₋₆, alcoxy en C₁₋₆, alkylthio en C₁₋₆, acyle en C₁₋₆, carboxyle, alcoxycarbonyle en C₁₋₆, hydroxyle, nitro, amino éventuellement N-mono- ou disubstitué par alkyle en C₁₋₄, acylamino en C₁₋₆, alcoxycarbonylamino en C₁₋₆, carbamoyle éventuellement N-mono- ou disubstitué par alkyle en C₁₋₄, cyano alkylsulfinyle en C₁₋₆, alkylsulfonyle en C₁₋₆, aminosulfonyle éventuellement N-mono- or disubstitué par alkyle en C₁₋₄, aminosulfonylamino N-mono- ou disubstitué par alkyle en C₁₋₄, aminosulfonylamino; Y est l'oxygène ou N - R₃ où R₃ est un hydrogène, un alkyle en C₁₋₆ ou un benzyle éventuellement substitué par un ou plusieurs alkoxy en C₁₋₆; A est un groupe choisi parmi: 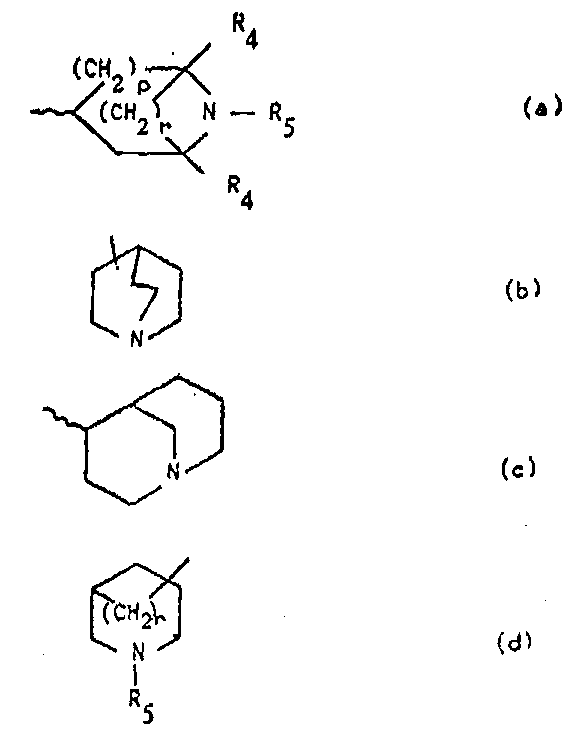 où p est 0, 1 ; r est 0, 1, 2, 3; R₄ est un atome d'hydrogène ou un groupe alkyle en C₁₋₄; R₅ est un atome d'hydrogène, un alkyle en C₁₋₆, un cycloalkyle en C₃₋₈, un cycloalkyle en C₃₋₈-alkyle en C₁₋₄, un alkyle en C₁₋₄, substitué par un phényle ou R₅ est un groupe de formule 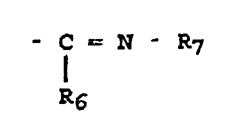 dans laquelle R₆ est un atome d'hydrogène, un alkyle en C₁₋₄ ou un groupe amino et R₇ est un atome d'hydrogène ou un alkyle en C₁₋₆, leurs tautomères et les sels d'addition d'acide desdits composés.

2. Sels d'addition d'acide physiologiquement compatibles des composés de formule générale (I) selon la revendication 1.

3. Sels selon la revendication 2, caractérisés en ce que les acides physiologiquement compatibles sont l'acide chlorhydrique, l'acide bromhydrique, l'acide sulfurique, l'acide acétique, l'acide citrique ou l'acide tartrique.

4. Composés de formule générale (I) selon la revendication 1, caractérisés en ce que A représente un groupe endo-8-méthyl-8-azabicyclo[3.2.1]oct-3-yle, R₁ et R₂ sont H, R est H ou CH₃ et Y est l'oxygène ou un groupe NH, leurs tautomères et les sels d'addition d'acide desdits composés.

5. Composés de formule générale (I) selon la revendication 1, caractérisés en ce que A représente un groupe endo-9-méthyl-9-azabicylo[3.3.1]non-3-yle, R₁ et R₂ sont H, R est H ou CH₃ et Y est l'oxygène ou un groupe NH, leurs tautomères et les sels d'addition d'acide desdits

6. Composés de formule générale (I) selon la revendication 1, caractérisés en ce que A représente un groupe endo-1-azabicyclo[3.3.1]non-4-yle, R₁ et R₂ sont H, R est H ou CH₃ et Y est l'oxygène ou un groupe NH, leurs tautomères et les sels d'addition d'acide desdits composés.

7. Composés de formule générale (I) selon la revendication 1, caractérisés en ce que A représente un groupe 1-azabicyclo[2.2.2]oct-3-yle, R₁ et R₂ sont H, R est H ou CH₃ et Y est l'oxygène ou un groupe NH, leurs tautomères et les sels d'addition d'acide desdits composés.

8. Sels d'addition d'acide physiologiquement compatibles des composés de formule générale (I) selon les revendications 4, 5, 6 ou 7.

9. Sels selon la revendication 8, caractérisés en ce que les acides physiologiquement compatibles sont l'acide chlorhydrique, l'acide bromhydrique, l'acide sulfurique, l'acide acétique, l'acide citrique et l'acide tartrique.

10. Composé selon la revendication 1, qui est la N-(1-azabicyclo[2.2.2]oct-3-yl)-2,3,dihydro-2-oxo-1H-benzimidazole-1-carboxamide.

11. Composé selon la revendication 1, qui est le N-(endo-9-méthyl-9-azabicyclo[3.3.1]non-3-yl)-3-méthyl-2,3-dihydro-2-oxo-1H-benzimidazole-1-carboxamide.

12. Composé selon la revendication 1, qui est le N-(endo-8-méthyl-8-azabicyclo[3.2.1]oct-3-yl)-2,3-dihydro-2-oxo-1H-benzimidazole-1-carboxamide.

13. Composé selon la revendication 1, qui est le N-(endo-9-méthyl-9-azabicyclo[3.3.1]non-3-yl)-2,3-dihydro-2-oxo-1H-benzimidazole-1-carboxamide.

14. Sels d'addition d'acide physiologiquement compatibles des composés selon la revendication 10, 11, 12 ou 13.

15. Sels selon la revendication 14, caractérisés en ce que les acides physiologiquement compatibles sont l'acide chlorhydrique, l'acide bromhydrique, l'acide sulfurique, l'acide acétique, l'acide citrique et l'acide tartrique.

16. Procédé de préparation des composés de formule générale (I) selon la revendications 1, dans lesquels R est H, caractérisé en ce qu'un composé de formule générale (II) 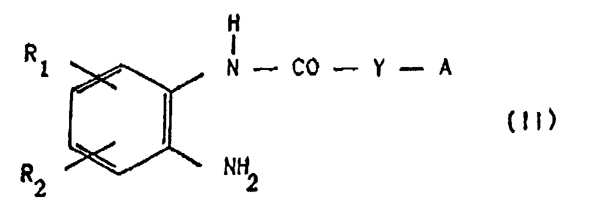 dans laquelle R₁, R₂, Y et A sont tels que définis dans la revendication 1, est mis à réagir avec un dérivé carbonylé réactif de formule générale (III) 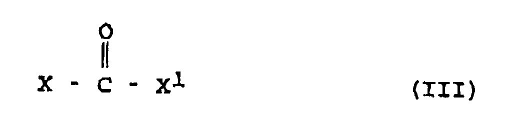 dans laquelle X et X¹ sont des groupes partants, identiques ou différents l'un de l'autre, dans un solvant aprotique à une température comprise entre 0°C et 100°C.

17. Procédé selon la revendication 16, caractérisé en ce que le groupe partant est choisi parmi le chlore, un groupe trichlorométhoxy, méthoxy, éthoxy ou imidazolyle.

18. Procédé de préparation des composés de formule générale (I) selon la revendications 1, dans lesquels R₁ et R₂ sont tous les deux des atomes d'hydrogène, caractérisé en ce qu'un composé de formule générale (VII) 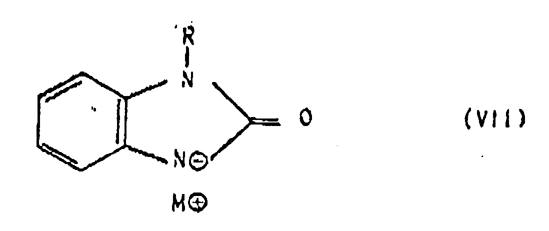 dans laquelle M est un atome métallique, est mis à réagir avec un intermédiaire réactif de formule générale (VI) 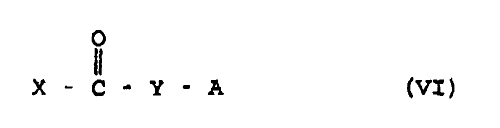 dans laquelle Y et A sont tels que définis dans la revendication 1 et X est tel que défini dans la revendication 16, dans un solvant aprotique polaire à une température comprise entre 0°C et 100°C.

19. Procédé selon la revendication 18, caractérisé en ce que l'atome métallique est choisi parmi le sodium, le potassium et le lithium.

20. Procédé de préparation des composés de formule générale (I) selon la revendication 1, dans lesquels R₁ et R₂ sont tous les deux des atomes d'hydrogène, caractérisé en ce qu'un dérivé réactif de formule (VIII) 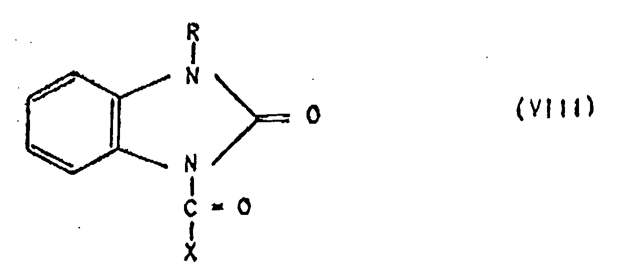 dans laquelle R est tel que défini dans la revendication 1 et X est tel que défini dans la revendication 16 est mis à réagir avec un composé de formule (IX)
Z - Y - A (IX)
dans laquelle Z représente un atome d'hydrogène, de lithium, de sodium ou de potassium, Y et A sont tels que définis dans la revendication 1, dans un solvant aprotique en présence d'un accepteur d'acide à une température comprise entre 0°C et 200°C.

21. Compositions pharmaceutiques comprenant comme ingrédient actif ou moins un composé de formule générale (I) selon la revendication 1 ou un tautomère ou des sels d'addition d'acide physiologiquement compatibles de celui-ci en association avec des véhicules ou excipients pharmaceutiques.

22. Compositions pharmaceutiques selon la revendication 21 en tant qu'agents 5-HT.

23. Compositions pharmaceutiques selon la revendication 21 utilisables dans le traitement des patients souffrant de nausées et de vomissements provoquées par la chimiothérapie et les rayonnements ou d'évacuation gastrique retardée, dans les troubles de la motilité gastro-intestinale et en particulier dans la dyspepsie, la flatulence, le reflux oesophagien, le syndrome du côlon irritable et l'hypokinésie.

24. Compositions pharmaceutiques selon la revendication 21 utilisables dans le traitement des patients souffrant de mal des transports, de migraine, de céphalées vasculaires de Horton, d'anxiété et de psychose.
